(19) Europäisches Patentamt
European Patent Office
Office européen des brevets

(11) Veröffentlichungsnummer: **0 004 956**
**B1**

(12) # EUROPÄISCHE PATENTSCHRIFT

(45) Veroffentlichungstag der Patentschrift: **14.10.81**

(21) Anmeldenummer: **79101153.9**

(22) Anmeldetag: **17.04.79**

(51) Int. Cl.³ **C 07 D 501/20,** C 07 D 499/64, A 61 K 31/545, A 61 K 31/43, A 23 K 1/17

(54) **Beta-Lactamantibiotika, Verfahren zu ihrer Herstellung, Futterzusatzmittel, Arzneimittel sowie Verfahren zu deren Herstellung.**

(30) Priorität: **26.04.78 DE 2818263**

(43) Veröffentlichungstag der Anmeldung: **31.10.79 Patentblatt 79/22**

(45) Bekanntmachung des Hinweises auf die Patenterteilung: **14.10.81 Patentblatt 81/41**

(84) Benannte Vertragsstaaten: **BE CH DE FR GB IT LU NL SE**

(56) Entgegenhaltungen:
**DE-A-1 770 031**
**DE-A-1 770 033**
**GB-A-2 000 139**

(73) Patentinhaber: **BAYER AG, Zentralbereich Patente, Marken und Lizenzen, D-5090 Leverkusen 1, Bayerwerk (DE)**

(72) Erfinder: **König, Hans-Bodo, Dr., Herberts Katernberg 10, D-5600 Wuppertal 1 (DE)**
Erfinder: **Metzger, Karl Georg, Dr., Pahlkestrasse 15, D-5600 Wuppertal 1 (DE)**
Erfinder: **Preiss, Michael, Dr., Egenstrasse 21, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

β-Lactamantibiotika, Verfahren zu ihrer Herstellung, Futterzusatzmittel, Arzneimittel, sowie Verfahren zu deren Herstellung

Die Erfindung betrifft β-Lactamverbindungen, Verfahren zu ihrer Herstellung sowie Arzneimittel, insbesondere antibakterielle Mittel und Mittel zur Förderung des Wachstums und zur Verbesserung der Futterverwertung bei Tieren.

β-Lactamverbindungen, die das Strukturelement

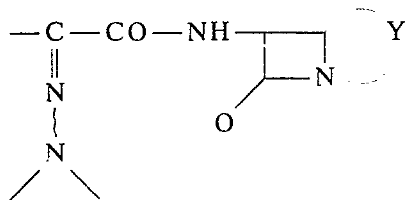

mit der unten beschriebenen Bedeutung von Y enthalten, sind in der japanischen Anmeldung 52 078 891 erwähnt, sie enthalten jedoch keine weitere heterocyclische Gruppe.

Es wurden nun neue β-Lactamverbindungen mit einem breiten antibakteriellen Spektrum gefunden, die der allgemeinen Formel I

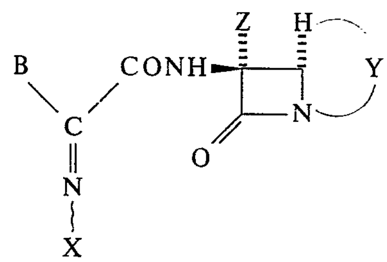

(I)

entsprechen, worin

B einen gegebenenfalls substituierten heterocyclischen 5- oder 6-Ring oder einen gegebenenfalls substituierten Phenylring

Z H oder $C_1-C_4$ Alkoxy

X ein Rest $-(NH-CO)_n-W$, worin n 0 oder 1 und W eine gegebenenfalls substituierte 2-Oxo-imidazolidin-1-yl-Gruppe, und

Y einen ein Penicillin- oder ein Cephalosporin-, ein Isocephem-, ein Des-thiaoxacephem- oder ein Isodes-thiaoxocephem-Derivat vervollständigenden Rest

bedeuten.

Geeignete heterocyclische 5- und 6gliedrige Reste B entsprechen den Formeln

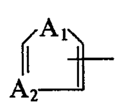

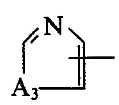

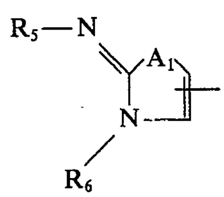

oder

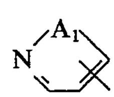

worin

$A_1$ NH, O oder S

$A_2$ CH oder N

$A_3$ CH = CH, CH = N oder N = CH bedeuten,

$R_5$ und $R_6$ unabhängig voneinander die Bedeutung von $R_1$ oder gemeinsam die Bedeutung von $E_3$ besitzen,

wobei die heterocyclischen Reste weitere Substituenten enthalten können, beispielsweise Amino,

Alkylamino, Dialkylamino, Alkyl, Hydroxy, $C_1-C_4$-Alkoxy, Acylamino oder einen Rest der Formel

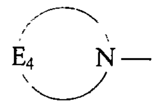

und $E_3$ und $E_4$ unabhängig voneinander einen zweiwertigen organischen Rest bedeuten,
und $R_1$ Wasserstoff, Alkyl, Alkenyl, Alkinyl, Alkadienyl, Cycloalkyl, Cycloalkenyl, Cycloalkadienyl, Aryl und Heterocyclyl bedeutet.

Geeignete gegebenenfalls substituierte Phenylreste B sind unsubstituierte Phenyl oder Phenyl 1- oder 2mal durch Hydroxy, Amino, Chlor, Fluor, Methoxy, Methyl, Acetoxy, Acetamino, Methylsulfonyloxy, Methylsulfonylamino, Hydroxysulfonylamino, Carboxy oder Aminocarbonyl substituiert. Bevorzugt sind Phenyl, Hydroxyphenyl und Aminophenyl.

Geeignete organische Reste Y entsprechen in Form der freien Säuren den Formeln

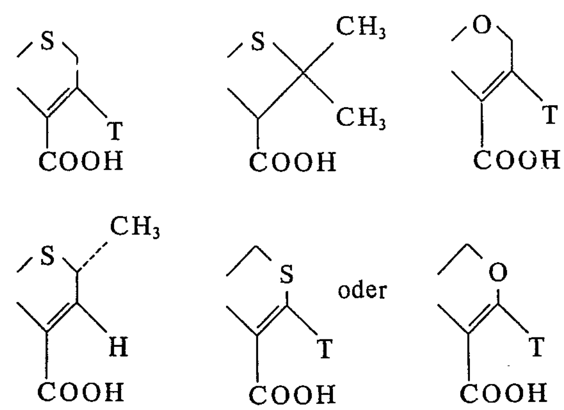

oder

worin

T Wasserstoff,

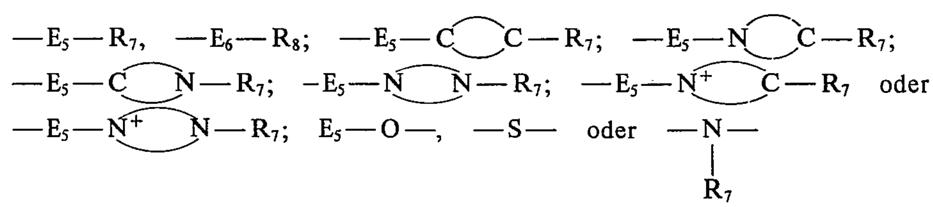

oder einen zweiwertigen organischen Rest, vorzugsweise

$$-(CH_2)_{1\text{ bis }4}, \quad -CH=, \quad -CH=CH-, \quad -(CH_2)_{1\text{ bis }2}CH=CH-,$$
$$-CH_2-S-CH_2-, \quad -CH_2-O-, \quad -CH_2-S-, \quad -CH_2-NH-$$
$$\text{oder} \quad -CH_2-N(R_7)-$$

oder eine direkte Bindung

$$E_6-(CH_2)_{1\text{ bis }4}; \quad -CH=, \quad -CH=CH-, \quad -(CH_2)_{1\text{ bis }2}CH=CH-$$
$$\text{oder} \quad -CH_2-S-CH_2-$$

oder eine direkte Bindung,
$R_7$ Wasserstoff, gegebenenfalls substituiertes Alkyl, gegebenenfalls substituiertes Alkenyl, gegebenenfalls substituiertes Cycloalkyl, gegebenenfalls substituiertes Cycloalkenyl, Aryl, Heterocyclyl, Acyl, Carboxyl oder ein funktionelles Derivat davon wie $C_1-C_4$-Alkoxycarbonyl, Aminocarbonyl, Cyano oder Halogencarbonyl; Formyl, Trifluoromethyl oder
$R_8$,

$R_8$ Halogen, gegebenenfalls substituiertes Amino, Heterocyclyl gebunden durch N, Hydroxy, Mercapto, $C_1-C_6$-Alkylsulfonyl, $C_1-C_6$-Alkoxysulfonyl, $C_1-C_6$-Alkylaminosulfonyl, Heterocyclylsulfonyl gebunden durch N, $C_1-C_6$-Alkylsulfinyl, $C_1-C_6$-Alkoxysulfinyl, Di-$C_1-C_6$-alkylaminosulfinyl, Heterocyclylsulfinylamino gebunden durch N, $C_1-C_6$-Alkylthiosulfonyl, gegebenenfalls substituiertes Hydrazino, gegebenenfalls substituiertes Hydrazono oder gegebenenfalls substituiertes Hydroxylamino,

— einen zweiwertigen heterocyclischen Rest, gebunden durch N oder C, der ein mono- oder Bicyclus ist, 1 bis 5 Heteroatome aus der Gruppe O, N oder S enthält und gegebenenfalls substituiert ist, bedeuten.

Geeignete Substituenten $-E_5-R_7$ und $-E_6-R_8$ sind zum Beispiel Wasserstoff, Methyl, Äthyl, 2-Äthoxycarbonyläthyl, 2-Phenäthyl, 4-Aminocarbonylbenzyl, 2-Acetyl-2-äthoxycarbonyläthyl, Carboxy, Aminocarbonyl, Acetyl, Cyano, Formyl, Oximinomethyl, 2-Methylthioäthyl, 1-Bromäthyl, Chlor, Hydroxy, Methoxy, Formyloxy, Acetoxy, Methylsulfonyloxy, 2,1,3-Thiadiazol-4-yl-oxy, Amino, Acetylamino, Morpholin-1-yl, Dimethylamino, Methylsulfonylamino, Methylthio, Methylsulfinyl, Methylsulfonyl, 5-Methyl-1,3,4-thiadiazol-2-thio, Phenylthio, Trifluormethyl, Difluormethyl, Hydroxymethyl, Formyloxymethyl, Acetoxymethyl, Aminocarbonyloxymethyl, tert.-Butylcarbonyloxymethyl, Methoxymethylcarbonyloxymethyl, Aminomethylcarbonyloxymethyl, Methoxymethyl, Allyloxymethyl, Phenoxymethyl, Dimethylaminomethyliminocarbonyloxymethyl, Formylaminocarbonyloxymethyl, Ureidocarbonyloxymethyl, (3-Hydroxy-4-carboxyphenyl)-aminocarbonyloxymethyl, (2-Carboxyphenyl)-carbonyloxymethyl, (2-Äthoxycarbonylaminophenyl)-carbonyloxymethyl, (2-Äthoxycarbonylaminosulfonylphenyl)-carbonyloxymethyl, Acetylmethylcarbonyloxymethyl, (2-Carboxyäthyl)-carbonyloxymethyl, α-Hydroxybenzylcarbonyloxymethyl, [1-(4-Chlorphenylthio)-2-carboxyäthyl]-carbonyloxymethyl, 1,3,4-Thiadiazol-2-yl-carbonylthiomethyl, 2,1,3-Oxadiazol-4-yl-carbonylthiomethyl, (4-Sulfophenyl)-thiomethyl, Pyrolidin-1-yl-thiocarbonylthiomethyl, Natriumthiosulfatomethyl, Äthoxythiocarbonylthiomethyl, (4-Methylpiperazin-1-yl)-thiocarbonylthiomethyl, Methylthiomethyl, Isopropylcarbonylthiomethyl, Sulfomethyl, 1,2,3,4-Oxatriazol-5-yl-methylthiomethyl, Vinyl, 1-Äthoxycarbonylvinyl, 3-Hydroxyphenyl, 3-Acetoxypropenyl, 3-Aminocarbonyloxypropinyl, 3-(2-Methyl-1,3,4-thiadiazol-5-yl-thio)-propanyl, Cyanomethyl, Carboxymethyl, Methoxycarbonylmethyl, Aminocarbonylmethyl, Dimethylaminocarbonylmethyl, (4-Methylpiperazin-1-yl)-methyl, Acetylmethyl,

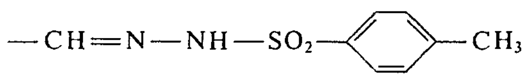

$$-CH=N-NH-CH_2-COOH$$

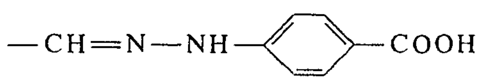

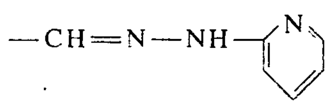

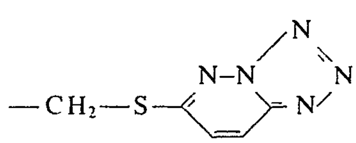

Geeignete Substituenten $-E_5-C$ $C-R_7$ sind zum Beispiel

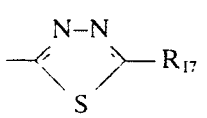
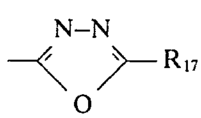

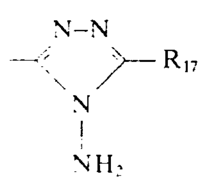
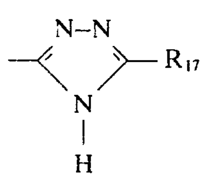

worin $R_{17}$ Wasserstoff, Methyl, Aminocarbonylmethylthio, 2-Hydroxyethylthio oder 2-Dimethylaminoethylthio bedeutet,

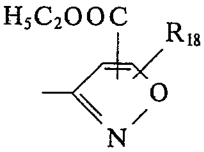

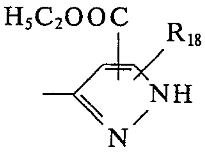

worin $R_{18}$ Ethoxycarbonyl, 4-Methylphenylsulfonyl, Cyano oder Phenyl bedeutet,

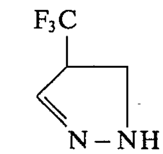

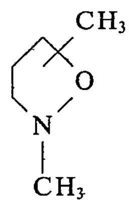

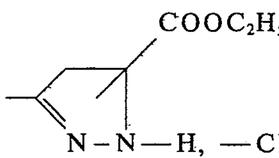, $-CH_3$

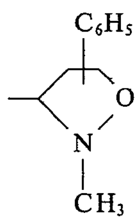

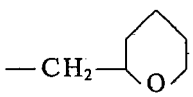

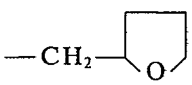

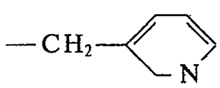

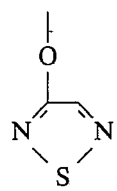

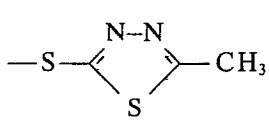

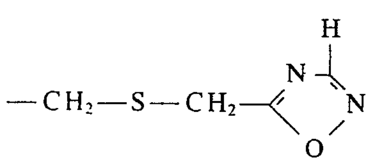

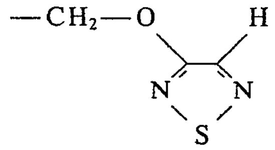

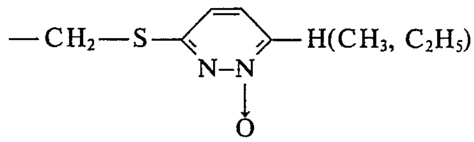

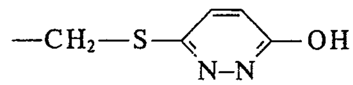

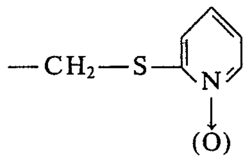

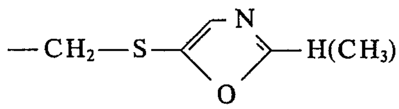

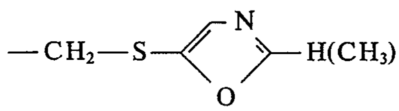

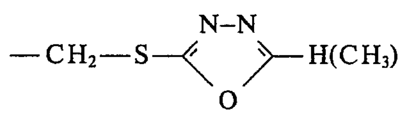

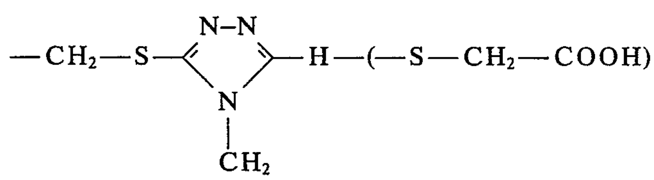

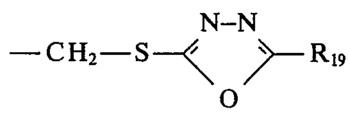

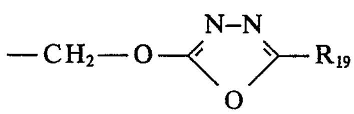

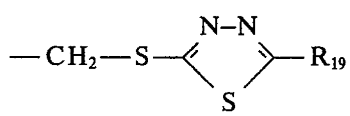

worin $R_{19}$ Wasserstoff, Methyl, Aminomethyl, Acetylaminomethyl, Guanidinocarbonylaminomethyl, Methylaminocarbonylaminomethyl, Methylsulphonylaminomethyl, 2-Aminoethyl, 2-Ureidoethyl, 2-Carbonylvinyl, Carbonylmethylthio, Sulpho, Aminosulfonyl, Acetylamino, Methylsulfonylamino, 4-Pyridino, 2-Dimethylaminoethylthio, 2-Hydroxyethylthio, Aminocarbonylmethylthio oder 2-Carboxyethylcarbonylamino bedeutet. Geeignete Substituenten $-E_5N \quad C-R_7$ sind zum Beispiel:

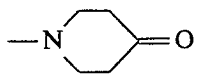

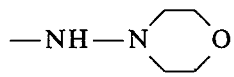

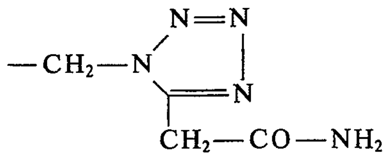

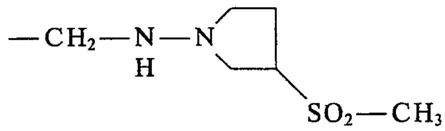

Geeignete Substituenten $E_5$—C N—$R_7$ sind zum Beispiel:

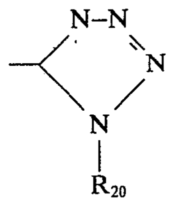

worin $R_{20}$ Wasserstoff, Methyl, 2-Dimethylaminoethyl, Carboxymethyl, Ethoxycarbonylmethyl, Aminocarbonylmethyl oder Sulphomethyl bedeutet,

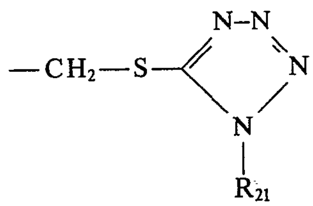

worin $R_{21}$ Wasserstoff, Methyl, $\omega$-Carboxy-$C_1$—$C_4$-alkyl, Sulphomethyl, 2-Aminoethyl, 2-Dimethylaminoethyl oder $\omega$-Sulphoamino-$C_2$—$C_5$-alkyl bedeutet,

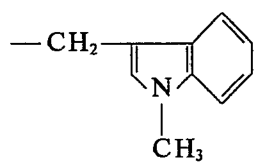

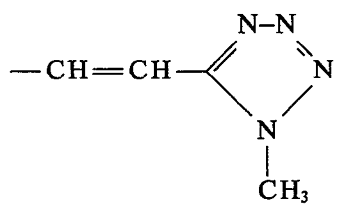

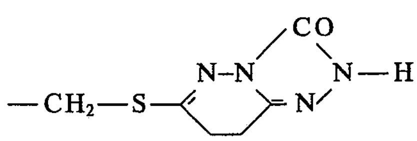

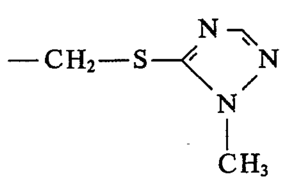

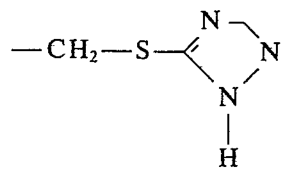

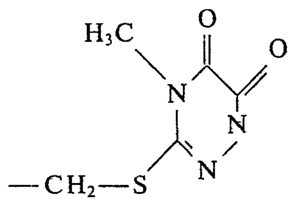

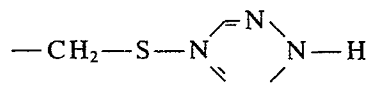

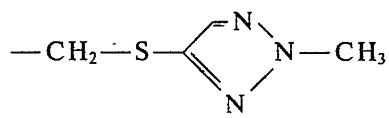

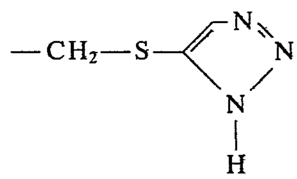

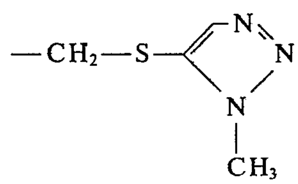

Ein geeigneter Rest $E_5-N$ $N-R_7$ ist zum Beispiel:

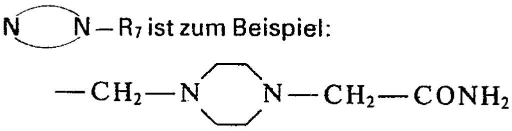

Geeignete Reste $R_5-N^+$ $C-R_7$ und $E_5-N^+$ $N-R_7$ sind zum Beispiel

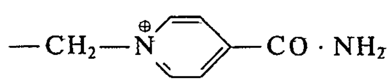

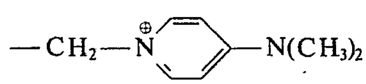

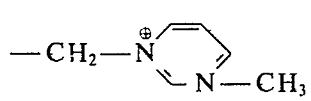

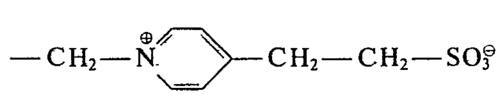

Geeignete Reste $E_3$ sind beispielsweise 2- oder 3gliedrige, gegebenenfalls durch Methyl, Äthyl, Cyclopropyl, Methylsulfonyl, Furylidenamino, Hydroxy, Oxo oder Amino substituierte Brücken, deren Brückenglieder Kohlenstoff- oder Stickstoffatome sein können.

Geeignete Reste $R_4$ sind z. B.

$-CO-(CH_2)_3-$, $-CO-(CH_2)_4-$, $-CONR_{12}-(CH_2)_2-$, $CONR_{12}-(CH_2)_3-$, $-(CO)_2-NR_{12}-(CH_2)_2$ oder $-(CH_2)_2-R_{10}-(CH_2)_2-$,

wobei

$R_{12}$ Wasserstoff, Methyl, Äthyl, Cyclopropyl, Methylsulfonylamino oder Furylidenamino bedeutet und
$R_{10}$ die bereits genannte Bedeutung hat.

Alkyl, $R_1$, $R_7$ als Substituent von B in der Definition Alkyl, Alkylamino und Dialkylamino ist insbesondere $C_1-C_4$-Alkyl, das durch Aryl, Heterocyclyl, Halogen, insbesondere Chlor und Brom, Hydroxy, $C_1-C_4$-Alkoxy, Acyloxy oder Acylamino substituiert sein kann, vorzugsweise Methyl und Äthyl. Alkenyl und Alkinyl $R_1$ und $R_7$ enthalten insbesondere 2 bis 4 C-Atome.

Alkadienyl, Cycloalkenyl und Cycloalkadienyl $R_1$ und $R_7$ enthalten insbesondere 5—7 C-Atome. Cycloalkyl $R_1$ und $R_7$ ist insbesondere $C_3-C_6$-Cycloalkyl, vorzugsweise Cyclopropyl.

Aryl, $R_1$, $R_7$ und als Substituent des Alkylrests $R_1$ ist insbesondere durch $C_1-C_4$-Alkyl, $C_1-C_4$-Alkoxy oder Halogen, vorzugsweise Chlor oder Brom, substituiertes Phenyl.

Heterocyclyl, $R_1$ und $R_7$ und als Substituent des Alkylrests ist insbesondere Thienyl, Thiazolyl, Pyridyl, Pyrrolidin-2-on-1-yl, Imidazolidin-2-on-1-yl, in der 3-Stellung durch $CH_3$, $C_2H_5$, Cyclopropyl, Mesyl, Acetyl, Methylaminosulfonyl oder Furylidenamino substituiertes Imidazolidin-2-on-1-yl oder Piperazin-2,3-dion-1-yl ist.

Acyl. $R_1$, $R_7$, in der Bedeutung Acylamino, als Substituent von B oder des Alkylrests $R_1$ sowie in der Bedeutung Acyloxy als Substituent des Alkylrests $R_1$ entspricht insbesondere der Formel

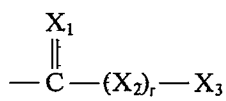

wobei

$X_1$ und $X_2$ unabhängig voneinander O, S oder $N-X_3$
$X_3$ $C_1-C_4$-Alkyl, $C_3-C_7$-Cycloalkyl, $C_2-C_4$-Alkenyl, Aryl oder Heterocyclyl und
r 0 oder 1 bedeuten, wobei Aryl und Heterocyclyl die bereits bekannte Bedeutung besitzen,

oder der Formel

$$-SO_2-X_4$$

wobei

$X_4$ $C_1-C_4$-Alkyl, Phenyl, $NH_2$, Mono- oder Dialkylamino oder Hydroxy bedeuten.

Die Verbindungen der Formel I können als freie Säure, Salz, insbesondere Natriumsalz, als inneres Salz oder als Ester vorliegen. Die =N—X-Bindung kann syn-

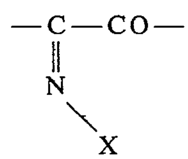

oder anti-ständig

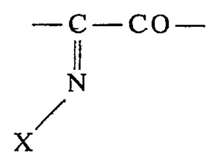

sein.

Bevorzugte Verbindungen entsprechend der Formel II

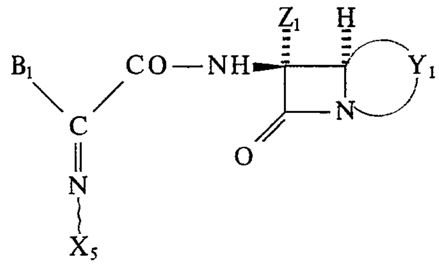

(II)

worin

$Y_1$ in Form der freien Säure einen Rest der Formeln

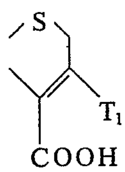

oder

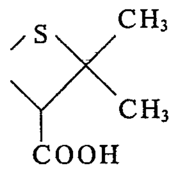

$T_1$

$CH_2—O—CO—CH_3$, $—CH_2—O—CO—NH_2$,

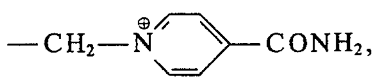

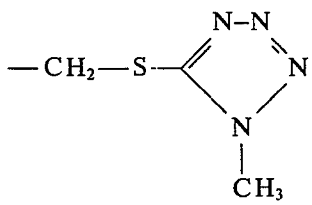

oder

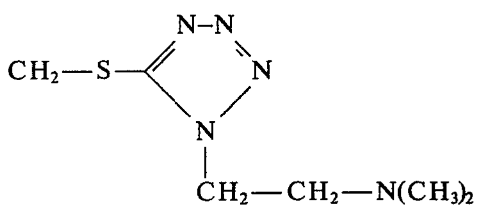

$Z_1$ Wasserstoff oder Methoxy

$B_1$ einen Rest der Formeln

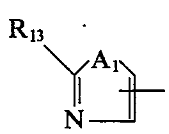

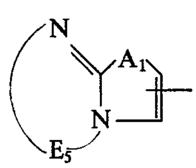

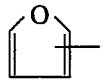

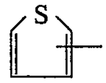

oder

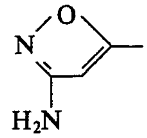

$X_5$

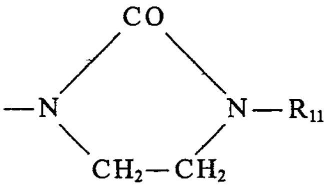

$R_{11}$ Wasserstoff, Methyl, Äthyl, Cyclopropyl, Methylsulfonyl oder Furylidenamino

$R_{13}$ Wasserstoff, Amino, $C_1-C_4$-Alkylamino, Di-$C_1-C_4$-alkylamino, $C_1-C_4$-Alkylcarbonylamino, Piperidino, Morpholino, Thiomorpholino, Piperazino, 4-$C_1-C_4$-Alkylpiperazino, 4-Oxothiomorpholino oder 4,4-Dioxothiomorpholino

$E_5$ $CH_2-CH_2$, $CH_2-CH_2-CH_2$, $CH=CH$, $CH=CH-CO-CO-CH=CH$, $CO-CH_2-CH_2$, $CH_2-CH_2-NH$, oder $NH-CH_2-CH_2$, wobei die CH- und $CH_2$-Reste durch OH oder $NH_2$ substituiert sein können.

Ganz besonders bevorzugte Verbindungen entsprechen der Formel III

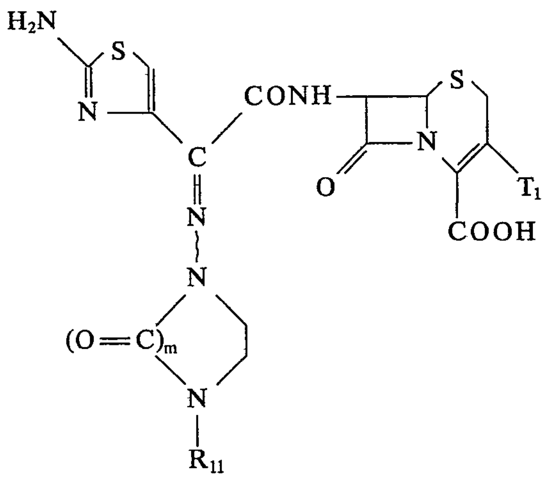

(III)

worin $T_1$, $R_{11}$ und m die bereits genannte Bedeutung haben.

Die jeweiligen syn- und anti-Formen ein und derselben Substanz sind in der Regel unterschiedlich wirksame Substanzen. Je nach Herstellungsweg und gegebenenfalls nach Art der Schutzgruppen kann bevorzugt die syn- oder anti-Form oder ein Gemisch beider Formen, mit gegebenenfalls überwiegenden Anteilen der einen Form gegenüber der anderen Form, entstehen. Auch ist es möglich, die eine Form in die andere teilweise oder ganz umzuwandeln, wobei sich Lewissäuren oder Protonensäuren als wirksam erwiesen haben. Die syn- und anti-Formen unterscheiden sich auch in ihren Spektren (z. B. NMR, IR) und in den Rf-Werten bei der Dünnschichtchromatographie. Man kann deshalb auch beide Formen durch chromatographische Verfahren präparativ voneinander trennen.

Die erfindungsgemäßen Verbindungen werden nach einer Reihe unterschiedlicher Verfahren erhalten, wobei die Verfahrensschritte an sich die gleichen sind, jedoch in unterschiedlicher Reihenfolge eingesetzt werden. Es hat sich gezeigt, daß die beiden folgenden Verfahrensvarianten am zweckmäßigsten sind. Nach Verfahren (1) setzt man die Verbindung der Formel IV

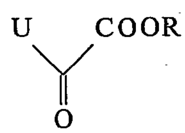

(IV)

worin

R Wasserstoff oder $C_1-C_4$-Alkyl und

U B, eine durch eine oder mehrere Schutzgruppen versehene Gruppe B oder eine chemische Vorstufe von B bedeuten;

erforderlichenfalls nach alkalischer Verseifung der Estergruppe mit Verbindungen der Formel V

$$H_2N-X \qquad (V)$$

worin X die bereits angegebene Bedeutung hat,

und anschließend, nach Aktivierung der Carbonylgruppe beispielsweise durch Umsetzung mit Chlorameisensäurealkylestern, mit Verbindungen der Formel VI

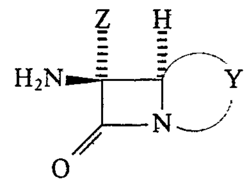

(VI)

worin Y und Z die obengenannte Bedeutung besitzen, um.

Gegebenenfalls schließt die Abspaltung der Schutzgruppen bzw. die Umwandlung der chemischen Vorstufe U in den Rest B an.

Nach Verfahren (2) wird die Verbindung IV mit der Verbindung VI beispielsweise in Gegenwart eines Wasser abspaltenden Mittels und einer Base umgesetzt. Anschließend wird in beliebiger Reihenfolge erforderlichenfalls der Rest B von Schutzgruppen befreit oder aus der Vorstufe U hergestellt und die Umsetzung mit der Verbindung V vorgenommen.

Ferner wurde gefunden, daß man die Verbindungen der Formel I auch dadurch erhalten kann, daß man Verbindungen der allgemeinen Formel I, in denen T die Bedeutung von $-CH_2-O-CO$-Niederalkyl, insbesondere von $-CH_2-O-CO-CH_3$ hat, mit Nukleophilen, beispielsweise mit

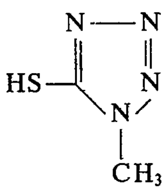

oder

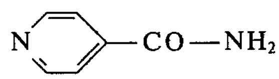

umsetzt.

Verbindungen der Formel I erhält man weiterhin, wenn man Verbindungen der allgemeinen Formel I, in denen T die Bedeutung von $-CH_2OH$ hat, mit $O=C=N-SO_2Cl$ oder $O=C=N-CO-CCl_3$ umsetzt, anschließend die $-SO_2-Cl$- oder $-CO-CCl_3$-Gruppe abspaltet und gegebenenfalls Schutzgruppen entfernt. Der Rest T ($-CH_2OH$) wird dadurch in an sich bekannter Weise in den Rest $T=CH_2-O-CO-NH_2$ umgewandelt.

Verbindungen der Formel I kann man auch erhalten, wenn man Verbindungen der Formel I, in denen Z Wasserstoff bedeutet und die in Form der freien Säuren, der Salze oder spaltbaren Ester vorliegen, in Gegenwart eines Methanolates, beispielsweise $Li-O-CH_3$, mit einem Hypochlorit, beispielsweise $(CH_3)_3C-O-Cl$ umsetzt und gegebenenfalls Schutzgruppen entfernt.

Verbindungen der allgemeinen Formel I, die in der syn-Form vorliegen, kann man auch dadurch erhalten, daß man Carbonsäuren der Formel VII in der syn-Form

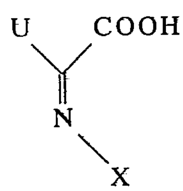

(VII)

in Zwischenverbindungen der allgemeinen Formel VIII

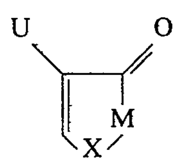

(VIII)

überführt,

worin M O, S oder NH bedeutet.

Dazu ist es nötig, daß im Rest X sich ein Sauerstoff-, Stickstoff- oder Schwefelatom in einer solchen Position befindet, daß die Carboxylgruppe oder Carbonsäure VII damit, gegebenenfalls nach Aktivierung, nach Art einer intramolekularen Cyclisierungsreaktion reagieren kann.

Ferner ist Voraussetzung, daß die Carboxylgruppe der Carbonsäure VII in der Zwischenverbindung VIII in einer ausreichend aktivierten Form vorliegt. Die Zwischenverbindung VIII muß nicht isolierbar

sein oder isoliert werden.

Durch Reaktion der Zwischenverbindung VIII mit der Aminoverbindung VI erhält man dann die Verbindung der allgemeinen Formel I in der syn-Form.

Die erfindungsgemäßen Verbindungen zeigen ein breites antibakterielles Spektrum gegen gram-positive und gram-negative Keime, insbesondere gegen Enterobakteriaceae, vor allem gegen solche mit $\beta$-Lactamasebildung.

Ferner verbessern die erfindungsgemäßen Verbindungen das Wachstum und die Futterausnutzung bei Tieren und sind als Antioxidantien verwendbar.

Die erfindungsgemäßen Wirkstoffe weisen bei geringer Toxizität eine starke und breite antimikrobielle Wirksamkeit auf. Diese Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z. B. Polymeren, Schmiermitteln, Farben, Fasern, Leder, Papier und Holz, von Lebensmitteln und von Wasser.

Mit Hilfe der erfindungsgemäßen Wirkstoffe können gramnegative und grampositive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Wirkstoffe gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z. B. Staphylococcus aureus, Staph. epidermidis, Staph. aerogenes (Staph. = Staphylococcus);

Lactobacteriaceae, wie Streptokokken, z. B. Streptococcus pyogenes, $\alpha$- bzw. $\beta$-hämolysierende Streptokokken, nicht ($\gamma$-)-hämolysierende Streptokokken, Str. viridans, Str. faecalis (Enterokokken), Str. agalactiae, Str. lactis, Str. anaero- bis und Diplococcus pneumoniae (Pneumokokken) (str. = Streptococcus);

Neisseriaceae, wie Neisserien, z. B. Neisseria gonorrhoeae (Gonokokken), N. meningitidis (Meningokokken), N. catarrhalis und N. flava (N. = Neisseria);

Corynebacteriaceae, wie Corynebakterien, z. B. Corynebacterium diphtheriae, C. pyogenes, C. diphtheroides, C. acnes, C. parvum, C. bovis, C. renale, C. ovis, C. murisepticum.

Enterobacteriaceae, wie Escherichiae-Bakterien der Coli-Gruppe: Escherichia-Bakterien, z. B. Escherichia coli, Enterobacter-Bakterien, z. B. E. aerogenes, E. cloacae, Klebsiella-Bakterien, z. B. K. pneumoniae, Serratia, z. B. Serratia marcescens (E. = Enterobacter) (K. = Klebsiella), Proteae-Bakterien der Proteus-Gruppe: Proteus, z. B. Proteus vulgaris, Pr. morganii, Pr. rettgeri, Pr. mirabilis, Providencia, z. B. Providencia sp. (Pr. = Proteus), Salmonelleae: Salmonella-Bakterien, z. B. Salmonella paratyphi A und B, S. typhi, S. enteritidis, S. cholerae suis, S. typhimurium (S. = Salmonella, Shigella-Bakterien, z. B. Shigella dysenteriae, Sh. flexneri, Sh. sonnei (Sh. = Shigella);

Pseudomonadaceae, wie Pseudomonas-Bakterien, z. B. Pseudomonas aeruginosa, (Ps. = Pseudomonas),

Parvobacteriaceae, wie Pasteurella-Bakterien, z. B. Pasteurella multocida, Haemophilus-Bakterien, z. B. Haemophilus influenzae, (H. = Haemophilus),

Bacteroidaceae, wie Bacteroides-Bakterien, z. B. Bacteroides fragilis, (B. = Bacteroides),

Bacillaceae, wie aerobe Sporenbildner, z. B. Bacillus anthracis (B. subtilis, B. cereus) B. = Bacillus), Anaerobe Sporenbildner-Chlostridien, z. B. Clostridium perfringens, (Cl. = Clostridium).

Die obige Aufzählung von Erregern ist lediglich beispielhaft und keineswegs beschränkend aufzufassen.

Als Krankheiten, die durch die erfindungsgemäßen Wirkstoffe verhindert, gebessert und/oder geheilt werden können, seien beispielsweise genannt:

Erkrankungen der Atmungswege und des Rachenraumes; Otitis; Pharyngitis; Pneumonie; Peritonitis; Pyelonephritis; Cystitis; Endocarditis; Systeminfektionen; Bronchitis; Arthritis; lokale Infektionen.

Zur vorliegenden Erfindung gehören pharmazeutische Zubereitungen, die neben nichttoxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten.

Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Die Wirkstoffe oder die pharmazeutischen Zubereitungen können lokal, oral, parenteral, intraperitoneal und/oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 5 bis etwa 1000, vorzugsweise 10 bis 150 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe, vorzugsweise in Mengen von etwa 1 bis etwa 250, insbesondere 10 bis 50 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Im Falle der Anwendung als Futterzusatzmittel können die neuen Verbindungen in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und ebenso eine Förderung des Wachstums und eine Verbesserung der Verwertung des Futters erreicht werden.

Die neuen $\beta$-Lactam-Antibiotika zeichnen sich durch starke antibakterielle Wirkungen, die in vivo und in vitro geprüft wurden, aus.

Die erfindungsgemäßen $\beta$-Lactam-Antibiotika können zum Zwecke der Erweiterung des Wirkungsspektrums und um eine Wirkungssteigerung zu erreichen auch mit Aminoglycosid-Antibiotika, wie Gentamicin, Kanamicin, Sisomicin, Amikacin, Tobramicin, Lactamaseinhibitoren wie Clavulansäure und Clavulansäurederivaten kombiniert werden.

Die Wirksamkeit der erfindungsgemäßen $\beta$-Lactamantibiotika kann durch die folgenden in vitro- und in vivo-Versuche beispielhaft demonstriert werden:

## 1. In vitro-Versuche

Die Verbindungen der Beispiele 5 und 9, welche als typische Vertreter der erfindungsgemäßen Verbindungen betrachtet werden können, wurden mit Müller-Hinton-Nährbrühe unter Zusatz von 0,1% Glucose auf einen Gehalt von 100 µg/ml verdünnt. In der Nährlösung befanden sich jeweils $1 \times 10^5$ bis $2 \times 10^5$ Bakterien pro Milliliter. Die Röhrchen mit diesem Ansatz wurden jeweils 24 Stunden bebrütet und anschließend wurde der Trübungsgrad bestimmt. Trübungsfreiheit gibt Wirkung an. Bei der Dosierung von 100 µg/ml waren die folgenden Bakterienkulturen trübungsfrei (sp. = species):

Klebsiella pneumoniae; Enterobacter aerogenes sp.; Providencia; Serratia marcescens; E. coli N; Salmonella sp.; Shigella sp.; Proteus, indolnegativ und indolpositiv; Staphylococcus aureus 133; Diplococcus pneumoniae sp.; Streptococcus pyogenes W.; Enterococcus sp.; Pseudomonas aeruginosa sp.

## Beispiel 1

### 2-(2-tert.-Butoxycarbonylimino-4-thiazolin-4-yl)-glyoxylsäureäthylester

10 g 2-(2-Aminothiazol-4-yl)-essigsäureäthylester werden in 30 ml tert.-Butanol bei 45°C tropfenweise mit 13,9 g Di-tert.-butylpyrocarbonat versetzt. Nach beendeter Umsetzung (Dünnschichtchromatogramm) wird das Lösungsmittel abgezogen und der Rückstand in Äther aufgenommen. Nach Waschen mit verd. Salzsäure, Trocknen und Einengen wird mit Petroläther ausgerieben (Schmelzpunkt nach Chromatographie: 78–79°C. 2,7 g dieser Substanz werden mit 2,1 g Selendioxid in 50 ml Dioxan, welches 1 ml Wasser enthält, 16 Std. unter Rückfluß gekocht. Dann wird einrotiert, der Rückstand mit Wasser und Essigester behandelt, die organische Phase einrotiert und der Rückstand mit Petroläther angerieben.

Ausbeute: 1,1 g.
Schmelzpunkt: 125° C.
NMR (100 MHz; δ in $CCl_4$): 8,06 (s; Thiazolin 5-H).

Beispiel 2

2-(2-tert.-Butoxycarbonylimino-4-thiazolin-4-yl)-glyoxylsäure

3,3 g der nach Beispiel 1 hergestellten Substanz werden in 15 ml Äthanol gelöst. Eine Lösung von 0,4 g Natriumhydroxyd in 15 ml Wasser wird zu dieser Lösung hinzugegeben und die Mischung dann 1 Stunde bei 20° C gerührt. Dann wird das Gemisch im Vakuum eingedampft, der Rückstand in Wasser gelöst, diese wäßrige Lösung mit Essigester gewaschen, mit verdünnter Salzsäure auf pH 1,5 angesäuert, der ausgefallene Niederschlag abgesaugt, mit Wasser gewaschen und getrocknet.

Ausbeute: 1,4 g.
Schmelzpunkt: ca. 150° C (Zers.).

Die Substanz ist nach dem Dünnschichtchromatogramm einheitlich.

NMR (100 MHz; δ in $d_6$-DMSO-$CDCl_3$): 8,24 (s, Thiazolin 5-H); 1,44 (s. $(CH_3)_3C$).

Beispiel 3

2-(2-tert.-Butoxycarbonylimino-4-thiazolin-4-yl)-N-(imidazolidin-2-on-1-yl)-2-iminoessigsäure

0,7 g 1-Amino-2-oxoimidazolidin werden in 5 ml Äthanol in der Wärme gelöst. Zu dieser Lösung gibt man 1,9 g der nach Beispiel 2 hergestellten Substanz hinzu und rührt die Mischung dann über Nacht bei 20° C. Der Niederschlag wird abgesaugt, mit Äthanol gewaschen und getrocknet.

Ausbeute: 1,3 g.
Schmelzpunkt: ca. 198° C (Zers.).
NMR (100 MHz; δ in $d_7$-DMF/$CD_3OD$): 7,34 (s; Thiazolin 5-H); 3,42 (»s«; $-CH_2-CH_2-$); 1,52 (s; $(CH_3)_3C$).

Die Substanz enthielt noch etwa 0,4 Moläquivalente Äthanol.

Beispiel 4

7-[2-(2-tert.-Butoxycarbonylimino-4-thiazolin-4-yl)-N-imidazolidin-2-on-1-yl)-2-iminoacetamido]-3-acetoxymethyl-3-cephem-4-carbonsäure

Mischung A

3,0 g der nach Beispiel 3 hergestellten Substanz werden in 40 ml Dichlormethan suspendiert. Bei 20° C werden dann 1,2 ml Triäthylamin zugegeben. Man rührt etwa 10 Minuten, kühlt dann auf −40° C und versetzt die Lösung nacheinander mit 0,05 ml 3-Dimethylaminopropanol und 0,81 ml Chlorameisensäureäthylester. Man rührt noch 20 Minuten bei −35° C und gibt 40 ml auf −35° C vorgekühltes Aceton zu.

Mischung B

3,0 g 7-Aminocephalosporansäure werden in 20 ml Wasser suspendiert und durch Zugabe der gerade eben nötigen Menge 1n-Natronlauge in Lösung gebracht. Man gibt 20 ml Aceton zu und kühlt auf −10° C.

Die Mischung B gibt man unter Rühren zur Mischung A und läßt bei 20° C Raumtemperatur 3 Stunden rühren. Dann wird mit viel Wasser verdünnt, Essigester zugegeben, durchgeschüttelt und eine kleine Menge Ungelöstes abgesaugt. Die wäßrige Phase wird abgetrennt und nochmals mit Essigester extrahiert. Die wieder abgetrennte wäßrige Phase wird dann mit frischem Essigester überschichtet und unter Rühren mit verdünnter Salzsäure bis pH 1,5 angesäuert. Die Essigester-Phase wird abgetrennt, mit etwas Wasser gewaschen, über Magnesiumsulfat getrocknet und im Vakuum

eingeengt. Dabei fällt ein Niederschlag (Fällung 1) aus, der abgesaugt wird (2,0 g). Das Filtrat dieses Niederschlages wird im Vakuum völlig eingedampft (Rückstand 1,5 g). Beide Substanzen werden getrennt in Dichlormethan gelöst bzw. suspendiert und über Nacht bei 20°C stehengelassen. Dabei kristallisiert die Substanz.

Gesamtausbeute: 2,0 g.
Schmelzpunkt: etwa 190°C (Zers.).
NMR (100 MHz; δ in $d_6$-DMSO/$CD_3OD$): 8,30 (s; Thiazolin 5-H); 5,83 (d; J 5,0 Hz; 7-H); 5,18 (d; J 5,0 Hz; 6-H); 5,08 und 4,80 (AB; J 13 Hz; 3-$CH_2-O$); 3,68 und 3,55 (AB; J 18 Hz; 2-$CH_2$); 3,30 (»s«; $-CH_2-CH_2-$); 2,04 (s; $-CO-CH_3$); 1,50 (s; $(CH_3)_3-C$).

## Beispiel 5

7-[2-(2-Amino-4-thiazo-4-yl)-N-(imidazolidin-2-on-1-yl)-2-iminoacetamido]-3-acetoxymethyl-3-cephem-4-carbonsäure

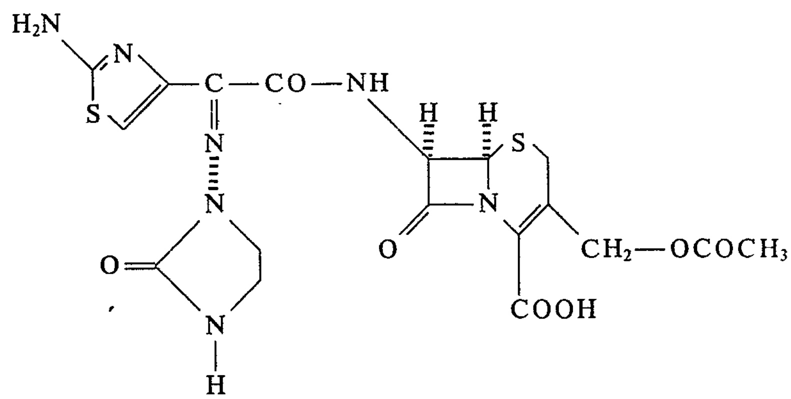

0,8 g der nach Beispiel 4 hergestellten Substanz werden mit 2,0 ml Anisol und, nach Abkühlung auf 0°C bis 5°C, mit 12 ml Trifluoressigsäure versetzt. Die Mischung wird bei der gleichen Temperatur 1 Stunde gerührt und dann in eine Mischung von Äther und Petroläther (8 : 2) eingegossen. Es bildet sich ein feiner Niederschlag. Er wird abfiltriert, mit Äther gewaschen, 2 Stunden in Essigester bei 20°C gerührt, wieder abfiltriert und getrocknet.

Ausbeute: 0,5 g des trifluoressigsauren Salzes.

Nach dem NMR-Spektrum ist die Substanz ein Gemisch der syn- und anti-Form und enthält noch 0,25 Moläquivalente des Ausgangsmaterials (Substanz von Beispiel 4).

Mischungsverhältnis der beiden isomeren Formen (a) und (b) ist etwa 1 zu 3 (nach NMR-Spektrum).

Form (a) des Gemisches wird angereichert, indem man als Ausgangsmaterial für die Entfernung der Schutzgruppe mit Trifluoressigsäure die der Fällung 1 im Beispiel 4 entsprechende Fraktion verwendet und diese Substanz (0,5 g) in 20 ml Wasser löst, mit Essigester überschichtet, den pH mittels Natronlauge von 2,6 auf 3,0 bringt, 1 Stunde bei 20°C rührt, eine kleine Menge ungelöste klebrige Substanz entfernt, die wäßrige Phase abtrennt, noch zweimal mit Essigester wäscht, mit Natronlauge auf pH 4,5 bringt, im Vakuum fast vollständig eindampft, durch Zugeben von Isopropanol die Substanz ausfällt, absaugt, mit Isopropanol wäscht und über $P_2O_5$ trocknet.

Ausbeute: 0,2 g.

Nach dem Dünnschichtchromatogramm (Kieselgel Merck, Butanol/Äthanol/Wasser (4 : 1 : 2)) enthält diese Substanz überwiegend Form (a).

IR-Spektrum (Nujol; Carbonylbereich): 1755, 1715, 1650 – 1600 breite Absorption, 1520 $cm^{-1}$.

Die beiden Formen werden durch Hochdruckflüssigkeitschromatographie getrennt

NMR (ppm, 60 MHz, $CD_3OD$):
Form (a) als Na-Salz: 6,95 (s, Thiazolin 5-H)
Form (b) als Na-Salz: 6,65 (s, Thiazolin 5-H)

Form (a) ist gegenüber Form (b) gegen E. coli 183/58 achtmal wirksamer.

Beispiel 6

1-Furylidenimino-2-oxo-3-hydrazinocarbonylimidazolidin

Die Mischung von 3,45 g Hydrazinhydrat und 21 ml 50%igem wäßrigen Methanol wird auf 60 C erwärmt. Dann werden unter Rühren im Laufe von 100 Minuten 3,0 g 1-Furylidenimino-2-oxo-3-chlorcarbonyl in vielen kleinen Portionen eingetragen. Nach 3 Stunden wird der Niederschlag abgesaugt, gewaschen und getrocknet.

Ausbeute: 1,7 g, Schmelzpunkt: 200° C.

Die Substanz gibt im Dünnschichtchromatogramm nur einen Fleck.

Beispiel 7

2-(2-tert.-Butoxycarbonylimino-4-thiazolin-4-yl)-N-[(1-furylidenamino-2-oxo-imidazolidin-3-yl)-carbonylamino]-2-iminoessigsäure

1,5 g der nach Beispiel 6 hergestellten Substanz werden mit 1,7 g der nach Beispiel 2 hergestellten Substanz in 25 ml Äthanol 6 Stunden auf 40–50° C erwärmt. Der Niederschlag wird abgesaugt und getrocknet.

Ausbeute: 2,4 g.
Schmelzpunkt: 205 C (Zers.).

Beispiel 8

7-{2-(2-tert.-Butoxycarbonylimino-4-thiazolin-4-yl)-N-[(1-furylidenamino-2-oxo-imidazolidin-3-yl)-carbonylamino]-2-iminoacetamido}-3-acetoxymethyl-3-cephem-4-carbonsäure

Mischung A

1,5 g der nach Beispiel 7 hergestellten Substanz werden in 20 ml Dichlormethan suspendiert. 0,42 ml Triäthylamin und 0,02 ml 3-Dimethylaminopropanol werden zugegeben. Die Mischung wird 45 Min. bei 20° C gerührt. Man kühlt dann auf –45° C und gibt 0,29 ml Chlorameisensäureäthylester und 20 ml Aceton hinzu. Man rührt dann 200 Minuten bei –25° C.

Mischung B

1,1 g 7-Aminocephalosporansäure werden in 10 ml Wasser mit der gerade eben nötigen Menge 1n Natronlauge gelöst. Dann werden noch 10 ml Aceton zugegeben. Die Mischung wird auf –5° C gekühlt.

Mischung A und Mischung B werden vereinigt. Man rührt und läßt die Temperatur langsam auf 20 C kommen. Nach 3 Stunden wird mit Wasser verdünnt, der pH mittels Natronlauge auf 7,5 gebracht, mit viel Essigester durchgeschüttelt, eine kleine Menge unumgesetzte 7-Aminocephalosporansäure entfernt, die wäßrige Phase abgetrennt, mit frischem Essigester überschichtet und mit verdünnter Salzsäure bis pH 1,5 unter Rühren angesäuert. Nach dem Trocknen und Abziehen der Essigesterlösung hinterbleibt die Substanz als freie Säure.

Ausbeute: 0,2 g.

Nach dem Dünnschichtchromatogramm enthält die Substanz etwas nach Beispiel 7 hergestelltes Ausgangsmaterial.

Beispiel 9

7-{2-(2-Amino-4-thiazol-4-yl)-N-[(1-furylidenamino-2-oxo-imidazolidin-3-yl)-carbonylamino]
2-iminoacetamido}-3-acetoxymethyl-3-cephem-4-carbonsäure

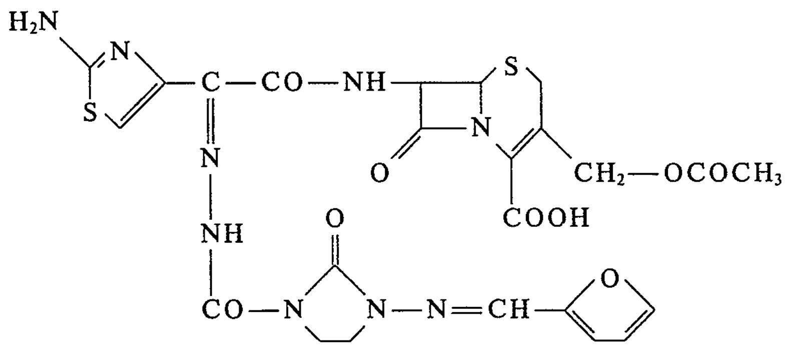

0,2 g der nach Beispiel 8 hergestellten Substanz werden mit 1,0 ml Anisol und 4,0 ml Trifluoressigsäure 15 Stunden bei 0° C – 5° C stehengelassen.

Die flüchtigen Teile werden im Hochvakuum abgezogen, der Rest wird mit Äther vertrieben und abgesaugt.

Ausbeute 0,2 g.

NMR (ppm, 60 MHz, $d_6$-DMSO + $CD_3OD$): 1,95 (3H, S, $CH_3CO$); 3,35 – 4,1 (6H, m, 2-$CH_2$, $-CH_2-CH_2$); 5,1 (1 H, d, 6-CH); 5,75 (1H, d, 7-CH); 6,4 – 6,6 (1H, m, 4-Furan); 6,8 (1H, d, 3-Furan); 6,95 (1H, s, Thiazolin 5-H); 7,7 (2H, s, breit, Furan 5-H, $-CH=N-$).

## Beispiel 10

7-[2-(2-tert.-Butoxycarbonylimino-4-thiazolin-4-yl)-2-oxoacetamido]-3-acetoxymethyl-3-cephem-4-carbonsäure

2,7 g 2-(2-tert.-Butoxycarbonylimino-4-thiazolin-4-yl)-glyoxylsäure, die in Moläquivalent Kristallalkohol enthält, wird in 40 ml Dichlormethan bei –30° C in Gegenwart von 2,8 ml Triäthylamin und 2 Tropfen 3-Dimethylaminopropanol mit insgesamt 2 ml Chlorameisensäureäthylester (in 3 Portionen zugegeben) aktiviert und dann mit der Lösung von 3,6 g 7-Aminocephalosporansäure in der Mischung von 40 ml Dichlormethan und 3,6 ml Triäthylamin vereinigt. Man läßt dann auf 20° C kommen, zieht nach 2 Stunden das Dichlormethan ab, wäscht die verbleibende wäßrige Lösung mit Essigester, säuert sie dann an (pH 1,5), extrahiert mit Essigester und dampft die getrocknete organische Phase im Vakuum ein. Der Rückstand wird mit Dichlormethan gewaschen und getrocknet.

Ausbeute: 1,8 g.

NMR (60 MHz; δ in $CD_3OD$): 8,35 (s, Thiazolin 5-H).

Die Substanz enthält teilweise keine BOC-Schutzgruppe mehr. Dadurch ist teilweise die nächste Reaktionsstufe vorweggenommen.

## Beispiel 11

7-[2-(2-Imino-4-thiazolin-4-yl)-2-oxoacetamido]-3-acetoxymethyl-3-cephem-4-carbonsäure

Die Lösung von 1,25 g der im Beispiel 10 hergestellten Substanz in 20 ml Trifluoressigsäure läßt man 1 Std. bei 20° C stehen, zieht dann entweder die Trifluoressigsäure ab und behandelt den Rückstand mit Äther oder versetzt das Reaktionsgemisch mit reichlich Äther. Der erhaltene Niederschlag wird abgesaugt und gründlich mit Dichlormethan gewaschen.

Ausbeute: 0,8 g.
NMR (60 MHz; δ in $CD_3OD$): 8,20 (s; Thiazolin 5-H).

Beispiel 12

7-[2-(2-Imino-4-thiazolin-4-yl)-N-(imidazolidin-2-on-1-yl)-2-iminoacetamido]-3-acetoxymethyl-3-cephem-4-carbonsäure

Man läßt die Lösung von 2,0 g 7-[2-(2-Imino-4-thiazolin-4-yl)-2-oxoacetamido]-3-acetoxymethyl-3-cephem-4-carbonsäure und 0,35 g 1-Aminoimidazolin-2-on in 60 ml 80%igem wäßrigem Tetrahydrofuran über Nacht bei 20°C stehen, filtriert und verdünnt das Filtrat mit Wasser. Dann wäscht man die Lösung mit Essigester, säuert die wäßrige Phase mit verdünnter Salzsäure an (pH 1,5) und schüttelt wieder mit Essigester aus. Die wäßrige Phase wird dann mit verdünnter Natronlauge neutralisiert und dann gefriergetrocknet.

Ausbeute: 2,1 g (Die Substanz enthält nach der Analyse 5,1 Moläquivalente NaCl).
NMR (60 MHz; $\delta$ in $D_2O$): 7,00 (s; Thiazolin 5-H).

Nach Reinigung durch Chromatographie des Natriumsalzes an Kieselgel zeigt die Substanz im Dünnschichtchromatogramm (Mikrobiologische Entwicklung; Testkeim: E. coli Neumann) nur einen Fleck, der bei gleicher Dosierung die gleiche Größe und Lage hat, wie die im Beispiel 5 beschriebene Substanz (Forma).

Beispiel 13

2-(2-Chloracetylimino-4-thiazolin-4-yl)-essigsäureäthylester

Zur Lösung von 40 g 2-(2-Aminothiazol-4-yl)-essigsäureäthylester in der Mischung von 400 ml Dichlormethan und 54 ml Pyridin gibt man nach Abkühlung auf 10°C 44,4 g Chloressigsäureanhydrid. Die Temperatur steigt auf etwa 20°C. Nach etwa 30 Minuten fällt ein Niederschlag aus. Man saugt ab und wäscht mit Dichlormethan.

Ausbeute: 30,6 g.
Schmelzpunkt: 141—42°C.

Aus der Mutterlauge lassen sich noch weitere 16,1 g Substanz (gleicher Schmelzpunkt) isolieren.

Beispiel 14

2-(2-Chloracetylimino-4-thiazolin-4-yl)-glyoxylsäureäthylester

Man gibt zur 50°C warmen Lösung von 160 g Selendioxid, 700 ml Dioxan und 31 ml Wasser die Mischung von 200 g der im Beispiel 13 beschriebenen Substanz und 1400 ml Dioxan. Es wird 19 Stunden unter Rückfluß gekocht. Nach Abkühlen auf 40°C wird vom Selen abfiltriert, die Mutterlauge mit Wasser verdünnt, das Dioxan im Vakuum größtenteils entfernt und die Substanz dann in viel Essigester aufgenommen. Die gewaschene und getrocknete Essigesterlösung wird dann einrotiert und der Rückstand aus Methanol umkristallisiert.

Ausbeute: 82,8 g.
Schmelzpunkt: 169—170°C.
IR-Spektrum (Nujol) (Carbonylbereich): 1730; 1700; 1680; 1540 $cm^{-1}$.

Beispiel 15

2-(2-Chloracetylimino-4-thiazolin-4-yl)-glyoxylsäure

Die Suspension von 18,5 g der Substanz von Beispiel 14 in der Mischung von 750 ml Äthanol und 300 ml Wasser wird durch kurzes Erwärmen und Abkühlen in eine Lösung übergeführt. Hierzu gibt man dann bei 23°C 134 ml 1n Natronlauge und läßt 10 Minuten stehen. Dann beginnt man den Alkohol abzurotieren. Nach 15 Minuten unterbricht man und stellt den pH mit verdünnter Salzsäure auf 7,5 und entfernt den restlichen Alkohol durch weiteres Abrotieren. Dann wird mit Essigester ausgeschüttelt und die abgetrennte wäßrige Phase mit verd. Salzsäure angesäuert (pH 1,5). Die dabei auskristallisierende Substanz wird abgesaugt, gewaschen und 2 Stunden bei 100°C im Vakuum getrocknet.

Ausbeute: 13,9 g.
Schmelzpunkt: ca. 217°C (Zersetzung).
NMR (100 MHz; $\delta$ in $CD_3OC/d_6$-DMSO): 8,38 (s; Thiazolin 5-H).

## Beispiel 16

### 2-(2-Chloracetylimino-4-thiazolin-4-yl)-N-(imidazolin-2-on 1-yl)-2-iminoessigsäure

2,0 g der im Beispiel 15 dargestellten Substanz und 0,81 g 1-Aminoimidazolin-2-on werden in 50 ml Äthanol kurz erwärmt und dann die Lösung 3 Tage bei 20° C stehengelassen. Die Substanz wird dann abgesaugt und bei 60° C im Vakuum getrocknet.

Ausbeute: ca. 260° C (Zersetzung).

Die Substanz ist ein Gemisch der syn- und anti-Formen.

NMR (100 MHz; $\delta$ in $CD_3OD/d_6$-DMSO): 7,27 s und 7,32 s (Thiazolin 5-H).

## Beispiel 17

### 6-[2-(2-Chloracetylimino-4-thiazolin-4-yl)-N-(imidazolidin-2-on-1-yl)-2-iminoacetamido]-penicillansäure

2,2- g der im Beispiel 16 beschriebenen Säure werden in der Mischung von 50 ml Dichlormethan und 0,92 ml Triäthylamin in Gegenwart von 0,03 ml 3-Dimethylaminopropanol bei −40° C mit 0,63 ml Chlorameisensäureäthylester aktiviert. Diese Lösung wird dann mit der auf 0° C abgekühlten Lösung von 1,86 g 6-Aminopenicillansäure in wäßrigem Aceton (mit 1 n-Natronlauge gelöst) vereinigt und die Mischung nach Entfernung des Kühlbades 3 Stunden gerührt. Dann wird mit reichlich Wasser verdünnt, mit Essigester ausgeschüttelt, die wäßrige Phase abgetrennt und dann nach Überschichtung mit frischem Essigester unter Rühren auf pH 1,5 angesäuert. Die organische Phase wird dann abgetrennt, gewaschen, getrocknet und einrotiert. Daher fällt ein Niederschlag aus. Man saugt ab und trocknet.

Ausbeute: 1,5 g.
NMR (100 MHz; $\delta$ in $CD_3OD$): 7,35 (s; Thiazolin 5-H), 4,3 (2 H, s, $Cl-CH_2-$), 3,3 (4 H, pseudo-s, Imidazolidinon)

1,66 (3 H, s, 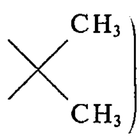), 1,52 (3 H, s, 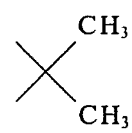)

## Beispiel 18

### 6-[2-(2-Imino-4-thiazolin-4-yl)-N-(imidazolidin-2-on-1-yl)-2-iminoacetamido]-penicillansäure

Die Mischung von 1,0 g der im Beispiel 17 hergestellten Substanz und 5 ml Dimethylformamid, 0,78 ml Triäthylamin und 0,22 g Thioharnstoff wird 20 Stunden bei 20°C gerührt. Dann wird das ausgeschiedene Triäthylaminhydrochlorid abgesaugt und aus dem Filtrat das Reaktionsprodukt mit Äther als Öl ausgefällt. Man gießt von dem Öl ab und behandelt es mit Isopropanol. Dabei wird es pulvrig. Man saugt ab, löst den Niederschlag dann in Wasser und säuert die wäßrige Lösung an (pH 2,0). Der daher ausfallende Niederschlag wird abgesaugt, mit Wasser gewaschen und getrocknet.

Ausbeute: 0,5 g.
IR-Spektrum (Nujol; Carbonylbereich): 1760; 1715; 1650-30 und 1540 $cm^{-1}$.
NMR (60 MHz; $\delta$ in $CD_3OD/d_7$-DMS): 7,3 (s; Thiazolin 5-H).

## Beispiel 19

### 7-[2-(2-Imino-4-thiazolin-4-yl)-N-(imidazolidin-2-on-1-yl)-2-iminoacetamido]-3-acetoxymethyl-3-cephem-4-carbonsäure

2,6 g einer entsprechend Beispiel 4 hergestellten Substanz werden in 50 ml Trifluoressigsäure 1 Stunde bei Raumtemperatur stehengelassen. Dann gibt man unter Kühlung mit Eis/Wasser ca. 300 ml Äther zu, saugt den ausgefallenen Niederschlag ab, wäscht gut mit Äther und trocknet.

Ausbeute: 2,1 g.

Nach dem NMR-Spektrum ist das Verhältnis der Form a zur Form b (siehe Beispiel 5) 1 zu 0,6.

Beispiel 20

1-Amino-2-oxo-3-methylimidazolidin

Diese Substanz erhält man durch Nitrosierung von 1-Methyl-2-oxo-imidazolidin in wäßriger Schwefelsäure und anschließende Reduktion mit Zinkstaub.

Siedepunkt (0,2 mm Hg): 77—84° C.

Beispiel 21

2-(2-tert.-Butoxycarbonylimino-4-thiazolin-4-yl)-N-(3-methylimidazolidin-2-on-1-yl)-2-iminoessigsäure

13,1 g 2-(2-tert.-Butoxycarbonylimino-4-thiazolin-4-yl)-glyoxylsäure werden in 160 ml Äthanol durch kurzes Erwärmen gelöst. Diese Lösung wird bei 20° C mit 5,5 g 1-Amino-2-oxo-3-methyl-imidazolidin versetzt. Nach Stehen über Nacht wird das Reaktionsgemisch im Rotationsverdampfer eingedampft, der Rückstand mit Wasser behandelt und der Niederschlag abgesaugt. Dieser Niederschlag wird mit überschüssiger $NaHCO_3$-Lösung versetzt und Ungelöstes abgesaugt. Das Filtrat wird angesäuert und der ausfallende Niederschlag abgesaugt, mit Wasser gewaschen und getrocknet.

Ausbeute: 11,3 g.
Schmelzpunkt: ab etwa 135° C (Zersetzung).
NMR (60 MHz; $\delta$ in $CD_3OD$): 7,2 (s, Thiazolin 5-H), 3,4 (pseudo-s, $-CH_2-CH_2$), 2,9 (s, $CH_3-N$).

Beispiel 22

7-[2-(2-tert.-Butoxycarbonylimino-4-thiazolin-4-yl)-N-(3-methylimidazolidin-2-on-1-yl)-2-iminoacetamido]-3-acetoxymethyl-3-cephem-4-carbonsäure

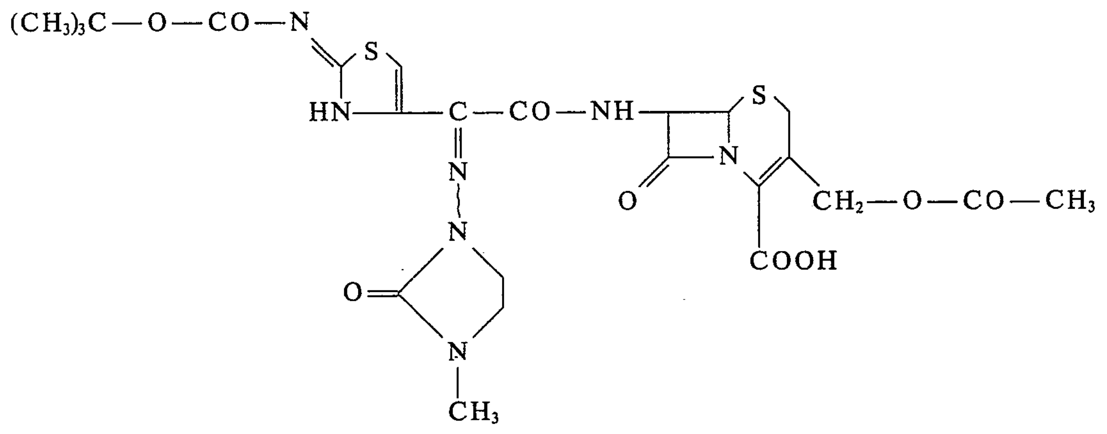

Mischung A

Zu der auf −40° C abgekühlten Lösung von 1 g des Produktes von Beispiel 21 in der Mischung von 30 ml Dichlormethan und 0,75 ml Triäthylamin gibt man 0,05 ml 3-Dimethylaminopropanol und 0,52 ml Chlorameisensäureäthylester.

Mischung B

Man löst 0,814 g 7-Aminocephalophoransäure in der Mischung von 20 ml Dichlormethan und 1 ml Triäthylamin unter Kühlung mit Eiswasser. Man gibt dann zur Mischung A die Mischung B hinzu und

rührt 2,5 Stunden bei 20 C. Das Reaktionsgemisch wird mit Wasser verdünnt und bei pH 7,5 zweimal mit Essigester ausgeschüttelt. Die wäßrige Phase wird mit frischem Essigester überschichtet, und bis pH 2 angesäuert. Die organische Phase wird abgetrennt, getrocknet, im Vakuum eingedampft und der Rückstand mit Äther behandelt. Der Niederschlag wird abgesaugt und getrocknet.

Ausbeute: 1,15 g.
NMR (60 MHz; δ in $CD_3OD$) 7,18 (s, Thiazolin 5-H) 2,9 (s, $CH_3-N$).

Beispiel 23

7-[(2-Imino-4-thiazolin-4-yl)-N-(3-methylimidazolidin-2-on-1-yl)-2-iminoacetamido]-3-acetoxymethyl-3-cephem-4-carbonsäure

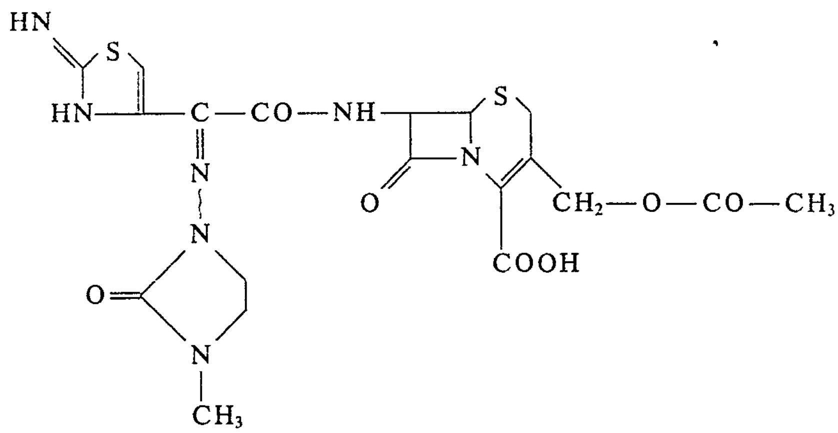

0,3 g des Produktes von Beispiel 22 werden unter Eiskühlung mit 4 ml Trifluoressigsäure versetzt. Man läßt die Mischung über Nacht bei 0 bis 5°C stehen und zieht dann die Trifluoressigsäure im Rotationsverdampfer ab. Der ölige Rückstand wird mit Äther verrieben, der Niederschlag wird abgesaugt, mit Äther gewaschen und getrocknet.

Ausbeute: 0,3 g des trifluoressigsauren Salzes.

Nach dem NMR-Spektrum ist die Substanz ein Gemisch der syn- und anti-Form.

NMR (ppm, 60 MHz, $CD_3OD$-$d_6$-DMSO): 6,8 und 6,87 (s, Thiazolin 5-H), 2,85 (s, $CH_3N$).

Beispiel 24

7-[2-(2-tert.-Butoxycarbonylimino-4-thiazolin-4-yl)-N-(imidazolidin-2-on-1-yl)-2-iminoacetamido]-3-(1-methyltetrazol-5-yl-thiomethyl)-3-cephem-4-carbonsäure

Mischung A

3,6 g der Substanz, hergestellt nach Beispiel 3, werden in 40 ml Methylchlorid suspendiert. 1,4 ml Triäthylamin werden bei 20°C zugegeben. Die Mischung wird 10 Minuten gerührt, dann auf −40°C gekühlt und mit 0,05 ml 3-Dimethylaminopropanol und 0,97 ml Chlorameisensäureäthylester versetzt. Die Mischung wird für 25 Minuten bei −40°C gerührt und dann auf −60°C gekühlt.

Mischung B

3,65 g 7-Amino-3-(1-Methyltetrazol-5-yl-thiomethyl)-3-cephem-4-carbonsäure werden in 40 ml Methylenchlorid suspendiert. 4,2 ml Triäthylamin werden zugegeben und die Mischung wird auf 0°C gekühlt.

Mischung B wird zu Mischung A gegeben, und die resultierende Mischung wird auf −60°C gekühlt. Dann wird die Mischung 3 3/4 Stunden bei 20°C gerührt und mit Wasser verdünnt. Das Methylenchlorid wird abgezogen und die verbleibende wäßrige Lösung wird mit Essigsäureäthylester gewaschen, dann auf pH 2 angesäuert und mit Essigsäureäthylester extrahiert. Die getrocknete

organische Phase wird im Vakuum auf 200 ml eingeengt. Ein gelber Niederschlag fällt aus, wird abfiltriert und mit Essigsäureäthylester gewaschen und getrocknet.

Ausbeute: 2,3 g.
IR-Spektrum (Nujol; Carbonylbereich): 1770, 1710 und 1540 $cm^{-1}$.
NMR (60 MHz; $\delta$ in $CD_3OD/CDCl_3$): 7,15 (s, Thiazolin 5-H), 4,0 (s., $CH_3$-Tetrazol).

Beispiel 25

7-[2(2-Aminothiazol-4-yl)-N-(imidazolidin-2-on-1-yl)-2-iminoacetamido]-3-(1-methyltetrazol-5-yl-thiomethyl)-3-cephem-4-carbonsäure

Die Mischung von 1,5 g der nach Beispiel 24 erhaltenen Substanz und 30 ml Trifluoressigsäure werden 45 Minuten bei 20° C stehengelassen. Die Mischung wird in 30 ml Diäthyläther gegossen. Der Niederschlag wird abfiltriert, mit Äther gewaschen und getrocknet.

Ausbeute: 1,15 g.
IR-Spektrum (Nujol; Carbonylbereich): 1770, 1720, 1670 und 1540 $cm^{-1}$.
MHK (Proteus vulg. 1017): 4 µg/ml.

Beispiel 26

2-Phenyl-N-(imidazolidin-2-on-1-yl)-2-iminoessigsäure

1,5 g Phenylglyoxylsäure wird in 25 ml 50%igem wäßrigem Äthanol gelöst. 1 g 1-Amino-2-oximidazolidin werden zu dieser Lösung gegeben, und die Mischung wird über Nacht bei 20° C gerührt. Der Niederschlag wird abfiltriert, mit Äthanol gewaschen und getrocknet.

Ausbeute: 2,2 g.
Schmelzpunkt: etwa 190° C (Zersetzung).
NMR (60 MHz; $\delta$ in $d_6$-DMSO): 7,6—7,2 (m; Phenyl); 3,4—2,8 (m; $-CH_2-CH_2$).

Beispiel 27

7-[2-Phenyl-N-(imidazolidin-2-on-1-yl)-2-iminoacetamido]-3-acetoxymethyl-3-cephem-4-carbonsäure

Mischung A

2,0 g der nach Beispiel 26 erhaltenen Substanz werden in 40 ml Methylenchlorid suspendiert. 1,19 ml Triäthylamin werden bei 20° C zugegeben. Die Mischung wird 10 Minuten gerührt und dann auf −20° C gekühlt. 0,05 ml 3-Dimethylaminopropanol und 0,82 ml Chlorameisensäureäthylester werden zu dieser Lösung gegeben. Die Mischung wird weitere 60 Minuten bei −20° C gerührt.

Mischung B

2,33 g 7-Aminocephalosporansäure werden in 50 ml Methylenchlorid bei 0° C suspendiert und mit 2,36 ml Triäthylamin versetzt.

Mischung B wird dann zu Mischung A gegeben, und die resultierende Mischung wird bei 20° C 4 Stunden gerührt. Die Reaktionsmischung wird mit Wasser verdünnt und durch zweimaliges Schütteln mit Essigsäureäthylester bei pH 7 extrahiert. Die wäßrige Phase wird mit einer frischen Lage von Essigsäureäthylester überschichtet und auf pH 2 angesäuert. Die organische Phase wird abgetrennt, getrocknet und im Vakuum eingedampft. Der Rückstand wird mit Äther behandelt. Der Niederschlag wird abfiltriert und getrocknet.

Ausbeute: 2,2 g (anti-Form?).
NMR (60 MHz; $\delta$ in $CD_3OD$): 7,5—7,2 (m; phenyl); 5,75 (d; 7-H); 2,05 (s; $-O-CO-CH_3$).
MHK (E/ml): E. coli 183/58: 8—16.

Beispiel 28

2-Phenyl-N-(imidazolidin-2-on-1-yl)-2-iminoessigsäure

5,0 g der nach Beispiel 26 erhaltenen Verbindung werden in 100 ml Methylenchlorid suspendiert und mit einer Lösung von 4,05 ml Thionylchlorid in 20 ml Methylenchlorid versetzt. Die Mischung wird über Nacht gerührt. Der Niederschlag wird abfiltriert, mit Methylenchlorid gewaschen und getrocknet.

Ausbeute: 1,75 g.
Schmelzpunkt: etwa 125° C (Zersetzung).

Beispiel 29

7-[2-Phenyl-N-(imidazolidin-2-on-1-yl)-2-iminoacetamido]-3-acetoxymethyl-3-cephem-4-carbonsäure

1,08 g 7-Aminocephalosporansäure werden in 20 ml Methylenchlorid suspendiert, und mit 1,0 ml Triäthylamin versetzt. Die Mischung wird 5 Minuten bei 0° C gerührt. Zu dieser Lösung werden 1 g der nach Beispiel 28 erhaltenen Substanz zugegeben. Die Mischung wird 3 Stunden bei 20° C gerührt, mit Wasser verdünnt und durch Schütteln mit Essigsäureäthylester extrahiert. Die wäßrige Phase wird auf pH 2 angesäuert und dann 3mal mit Essigsäureäthylester extrahiert. Die organische Phase wird abgetrennt, getrocknet und im Vakuum eingedampft. Der Rückstand wird mit Diäthyläther behandelt. Der Niederschlag wird abfiltriert und getrocknet.

Ausbeute: 2,2 g.

Die Lösung dieser Substanz in 5%iger wäßriger Natriumhydrogencarbonatlösung wird auf pH 2 angesäuert. Der Niederschlag wird abfiltriert, mit Wasser gewaschen und getrocknet.

Ausbeute: 0,6 g (syn-Form?).
NMR (60 MHz; $\delta$ in $CD_3OD$): 6,0 (d; 7-H); 2,05 (s; $-O-CO-CH_3$).
MHK (E/ml): E. coli 183/58: $\leq$ 0,25.

Beispiel 30

7-[2-(2-tert.-Butoxycarbonylimino-4-thiazolin-4-yl)-N-(imidazolidin-2-on-1-yl)-2-imino-acetamido]-3-[(2-methyl-1,3,4-thiadiazol-5-yl)-thio-methyl]-3-cephem-4-carbonsäure

Mischung A

1,9 g der nach Beispiel 3 erhaltenen Verbindung werden in 40 ml Methylenchlorid suspendiert und mit 0,8 ml Triäthylamin versetzt. Die Mischung wird auf −40° C gekühlt und mit 5 Tropfen 3-Dimethylaminopropanol und 0,53 ml Chlorameisensäureäthylester versetzt. Die Mischung wird 25 Minuten gerührt und auf −60° C gekühlt.

Mischung B

2,0 g 7-Amino-3-(2-methyl-1,3,4-thiadiazol-5-yl-thiomethyl)-3-cephem-4-carbonsäure werden in der Mischung von 40 ml Methylenchlorid und 2,4 ml Triäthylamin suspendiert und die Mischung wird auf 0° C gekühlt.

Mischung B wird zu Mischung A gegeben, und die resultierende Mischung wird auf −60° C gekühlt. Dann wird die Mischung 3,5 Std. bei 20°C gerührt. Sie wird dann mit Wasser verdünnt, das Methylenchlorid wird abgedampft, die wäßrige Lösung wird mit Essigsäureäthylester gewaschen, auf pH 1,8 angesäuert und mit Essigsäureäthylester extrahiert. Die getrocknete organische Phase wird vakuumstark eingeengt, und der Niederschlag wird abfiltriert und getrocknet.

Ausbeute: 1,4 g.
IR-Spektrum (Nujol): 1770, 1720, 1550 $cm^{-1}$.

Beispiel 31

7-[2(2-Aminothiazol-4-yl)-N-(imidazolidin-2-on-1-yl)-2-iminoacetamido]-3-[(2-methyl-1,3,4-thiadiazol-5-yl)-thiomethyl]-3-cephem-4-carbonsäure

Die Mischung von 0,7 g der nach Beispiel 31 erhaltenen Substanz und 14 ml Trifluoressigsäure werden 45 Minuten bei 20°C stehengelassen. Die Mischung wird dann in 140 ml Äther gegossen. Der Niederschlag wird abfiltriert, mit Äther gewaschen und getrocknet.

Ausbeute: 0,7 g.
IR-Spektrum (Nujol; Carbonylbereich): 1770, 1710, 1650 und 1550 $cm^{-1}$.
MHK (E. coli t 7): 0,25 E/ml

Beispiel 32

7-[2-(2-tert.-Butoxycarbonylimino-4-thiazolin-4-yl)-N-(imidazolidin-2-on-1-yl)-2-iminoacetamido]-3-[(2-methyl-1,3,4-oxidazol-5-yl)-thiomethyl]-3-cephem-4-carbonsäure

Mischung A

1,9 g der nach Beispiel 3 erhaltenen Substanz werden in 40 ml Methylenchlorid suspendiert und mit 0,8 ml Triäthylamin versetzt. Die Mischung wird auf −40°C gekühlt und mit 5 Tropfen 3-Dimethylaminopropanol und 0,53 ml Chlorameisensäureäthylester versetzt. Die Mischung wird 25 Minuten gerührt und dann auf −60°C gekühlt.

Mischung B

2 g 7-Amino-3-(2-methyl-1,3,4-oxdiazol-5-yl-thio-methyl)-3-cephem-4-carbonsäure werden in 40 ml Methylenchlorid suspendiert, auf 0°C gekühlt und mit 2,4 ml Triäthylamin versetzt.
Mischung B wird zu Mischung A gegeben, und die resultierende Mischung wird auf −60°C gekühlt. Dann wird die Mischung bei 20°C 3 Stunden gerührt, mit Wasser verdünnt und das Methylenchlorid abgezogen. Die wäßrige Lösung wird mit Essigsäureäthylester gewaschen, auf pH 1,8 angesäuert und mit Essigsäureäthylester extrahiert. Die getrocknete organische Phase wird im Vakuum stark eingeengt und der Niederschlag abfiltriert und getrocknet.

Ausbeute: 2,3 g.
IR-Spektrum (Nujol; Carbonylbereich): 1780, 1720 und 1550 $cm^{-1}$.

Beispiel 33

7-[2(2-Aminothiazol-4-yl)-N-(imidazolidin-2-on-1-yl)-2-iminoacetamido]-3-[(2-methyl-1,3,4-oxidazol-5-yl)-thiomethyl]-3-cephem-4-carbonsäure

Die Lösung von 1,0 g der nach Beispiel 32 erhaltenen Substanz werden in 20 ml Trifluoressigsäure 45 Minuten bei 20°C stehengelassen. Die Mischung wird in 200 ml Diäthyläther gegossen. Der Niederschlag wird abfiltriert, mit Diäthyläther gewaschen und getrocknet.

Ausbeute: 0,9 G.
IR-Spektrum (Nujol; Carbonylbereich): 1770, 1710, 1650, 1625 $cm^{-1}$.
MHK (E. coli Neumann): ≤ 1 E/ml.

In gleicher Weise kann folgende Verbindung hergestellt werden:

7-[2(2-Aminothiazol-4-yl)-N-(imidazolidin-2-on-1-yl)-2-iminoacetamido]-3-aminocarbonyloxymethyl-3-cephem-4-carbonsäure

Beispiel 34

6-[2-Phenyl-N-(imidazolidin-2-on-1-yl)-2-iminoacetamido]-penicillansäure

Mischung B

1,85 g 6-Aminopenicillansäure wird bei 0° C in 50 ml Methylenchlorid suspendiert und mit 4,72 ml Triäthylamin in Lösung gebracht. Diese Mischung wird bei 0° C zu Mischung A, hergestellt wie in Beispiel 27 beschrieben, gegeben, und die resultierende Lösung wird 3 Stunden bei 20° C gerührt. Das Reaktionsgemisch wird zweimal bei pH 7 mit Essigsäureäthylester extrahiert. Die abgetrennte wäßrige Phase wird dann bei pH 2 mit Essigsäureäthylester extrahiert, die organische Phase abgetrennt, getrocknet und im Vakuum konzentriert. Der ausgefallene kristalline Niederschlag wird abgesaugt und getrocknet.

Ausbeute: 2,15 g (vermutlich anti-Form?).
IR-Spektrum: (Nujol; Carbonylbereich) 1775, 1760, 1700, 1650 und 1510 $cm^{-1}$.
NMR (60 MHz; δ in $NaHCO_3/D_2O$): 7,7–7,1 (m; Phenyl), 5,7–5,4 (dd; 6-H, 5-H), 4,25 (s; 3-H), 3,4–2,9 (m; $N-CH_2-CH_2-N$), 1,65 und 1,55

s, s;

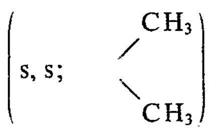

Die syn-Form wird aus der Substanz, die in Beispiel 28 beschrieben ist und 6-Aminopenicillansäure entsprechend Beispiel 29 hergestellt.

**Patentansprüche**

1. β-Lactamverbindungen der allgemeinen Formel I

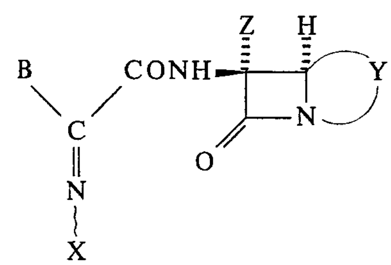

(I)

worin

B einen gegebenenfalls substituierten heterocyclischen 5- oder 6-Ring oder einen gegebenenfalls substituierten Phenylring
Z H oder $C_1-C_4$-Alkoxy
X ein Rest $-(NH-CO)_n-W$, worin n 0 oder 1 und W eine gegebenenfalls substituierte 2-Oxo-imidazolidin-1-yl-Gruppe, und
Y einen ein Penicillin- oder Cephalosporin-, ein Isocephem- ein Des-thiaoxacephem- oder ein Iso-des-thiaoxacephem-Derivat vervollständigenden Rest

bedeuten.

2. β-Lactamverbindungen gemäß Anspruch 1 der allgemeinen Formel II

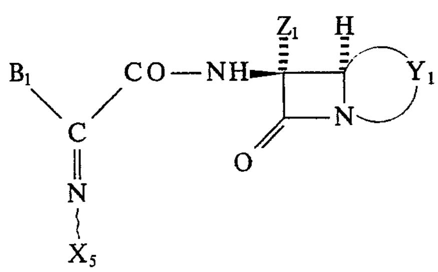

(II)

worin

$Y_1$ in Form der freien Säure einen Rest der Formeln

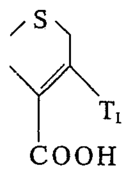

oder

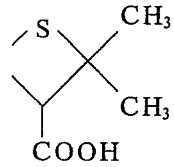

$T_1$

$CH_2—O—CO—CH_3$, $—CH_2—O—CO—NH_2$,

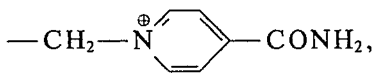

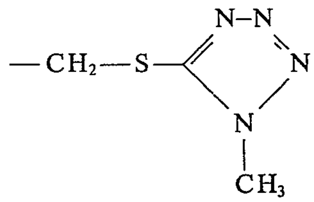

oder

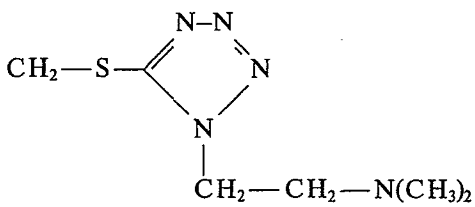

$Z_1$ Wasserstoff oder Methoxy

$B_1$ einen Rest der Formeln

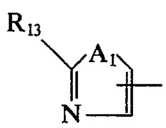

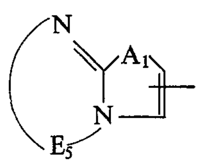

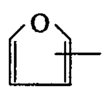

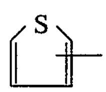

oder

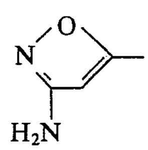

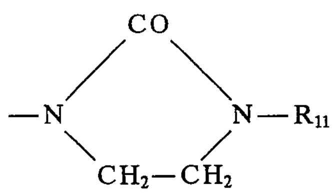

$X_5$

$R_{11}$ Wasserstoff, Methyl, Äthyl, Cyclopropyl, Methylsulfonyl oder Furylidenamino

$R_{13}$ Wasserstoff, Amino, $C_1—C_4$-Alkylamino, Di-$C_1—C_4$-alkylamino, $C_1—C_4$-Alkylcarbonylamino, Piperidino, Morpholino, Thiomorpholino, Piperazino, 4-$C_1—C_4$-Alkylpiperazino, 4-Oxothiomorpholino oder 4,4-Dioxothiomorpholino

$E_5$ $CH_2-CH_2$, $CH_2-CH_2-CH_2$, $CH=CH$, $CH=CH-CO-CO-CH=CH$, $CO-CH_2-CH_2$, $CH_2-CH_2-NH$, oder $NH-CH_2-CH_2$, wobei die CH- und $CH_2$-Reste durch OH oder $NH_2$ substituiert sein können, und

$A_1$ NH, O oder S

bedeuten.

3. β-Lactamverbindungen gemäß Anspruch 2 der allgemeinen Formel III

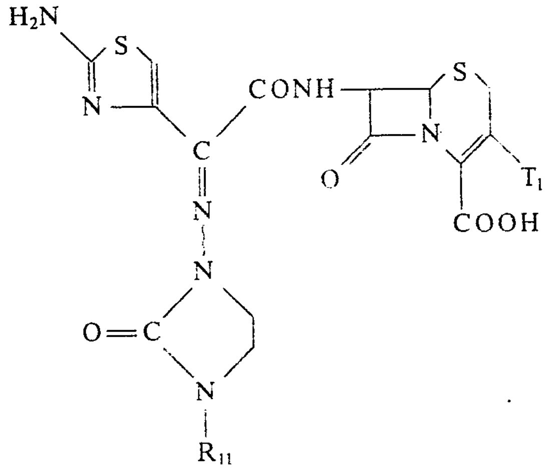

(III)

worin $T_1$, $R_{11}$ die in Anspruch 2 genannte Bedeutung haben.

4. 7-[2-Amino-4-thiazol-4-yl)-N-(imidazolidin-2-on-1-yl)-2-iminoacetamido]-3-acetoxymethyl-3-cephem-4-carbonsäure der Formel

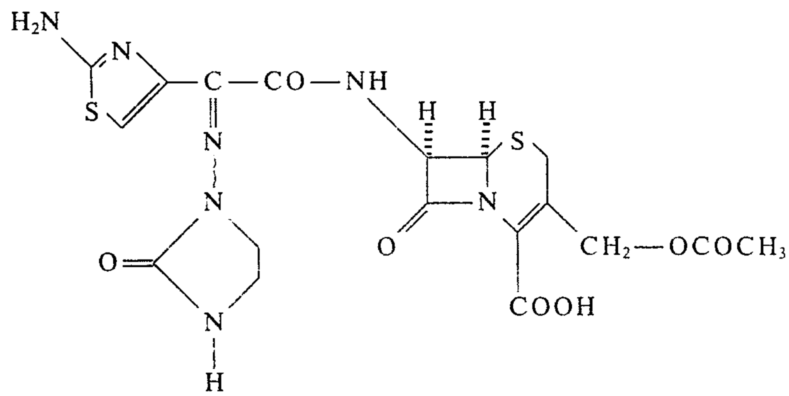

5. 7-{2-(2-Amino-4-thiazol-4-yl)-N-[(1-furylidenamino-2-oxo-imidazolidin-3-yl)-carbonylamino]-2-iminoacetamido}-3-acetoxymethyl-3-cephem-4-carbonsäure der Formel

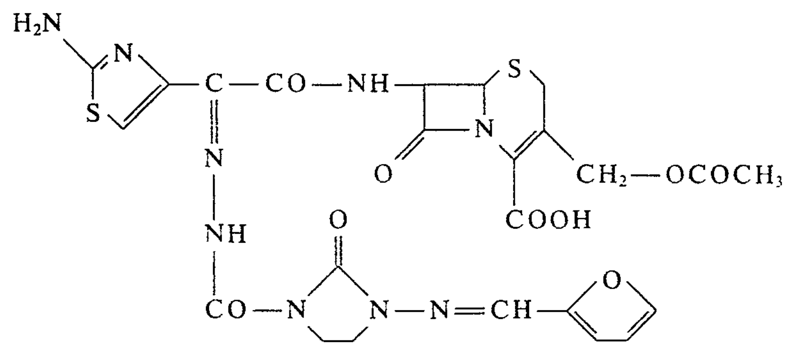

6. Verfahren zur Herstellung von β-Lactamverbindungen gemäß Anspruch 1, dadurch gekennzeichnet, daß man Verbindungen der Formel

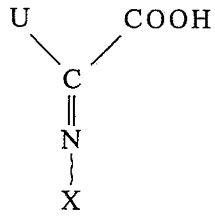

worin

U B, eine durch eine oder mehrere Schutzgruppen versehene Gruppe B oder eine chemische Vorstufe von B bedeutet und
B und X die in Anspruch 1 angegebene Bedeutung haben,

nach Aktivierung der Carbonylgruppe, mit Verbindungen der Formel

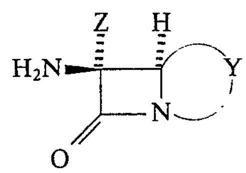

worin Z und Y die in Anspruch 1 genannte Bedeutung besitzen,

umsetzt und gegebenenfalls die Schutzgruppen abspaltet, oder die chemische Vorstufe U in B umwandelt.

7. Verfahren zur Herstellung von $\beta$-Lactamverbindungen gemäß Anspruch 1, dadurch gekennzeichnet, daß man Verbindungen der Formel

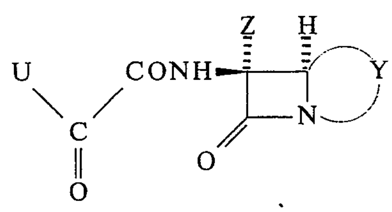

worin

U B, eine durch eine oder mehrere Schutzgruppen versehene Gruppe B oder eine chemische Vorstufe von B bedeutet und
Z und Y die in Anspruch 1 genannte Bedeutung besitzen,

mit Verbindungen der Formel

$$H_2N—X$$

worin X die in Anspruch 1 genannte Bedeutung besitzt,

umsetzt und gegebenenfalls Schutzgruppen abspaltet oder die chemische Vorstufe U in B überführt.

8. Arzneimittel, gekennzeichnet durch einen Gehalt an einer Verbindung gemäß Anspruch 1.

9. Verfahren zur Herstellung eines Arzneimittels, dadurch gekennzeichnet, daß man eine Verbindung gemäß Anspruch 1 mit pharmazeutisch geeigneten Träger- und/oder Zusatzstoffen vermischt.

10. Futterzusatzmittel, gekennzeichnet durch einen Gehalt an einer Verbindung gemäß Anspruch 1.

**Claims**

1. $\beta$-Lactam compounds of the general formula I

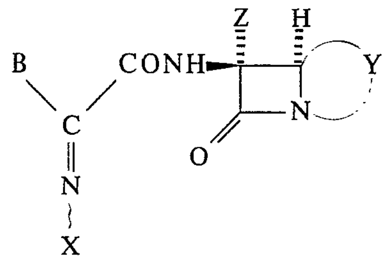

(I)

wherein

B denotes an optionally substituted heterocyclic 5-membered or 6-membered ring or an optionally substituted phenyl ring

Z denotes H or $C_1-C_4$ alkoxy

X denotes a radical $-(NH-CO)_n$ W, wherein n denotes 0 or 1 and W denotes an optionally substituted 2-oxoimidazolin-1-yl group, and

Y denotes a radical completing a derivative of penicillin or cephalosporin, isocephem, des-thiaoxacephem or iso-des-thiaoxacephem.

2. β-Lactam compounds according to Claim 1 of the general formula II

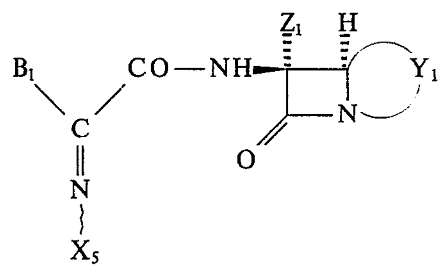

(II)

wherein

$Y_1$ in the form of the free acid, denotes a radical of the formulae

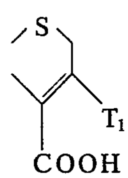

or

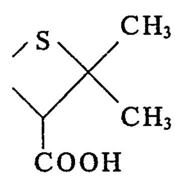

$T_1$ denotes

$$CH_2-O-CO-CH_3, \quad -CH_2-O-CO-NH_2,$$

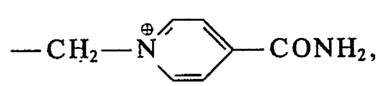

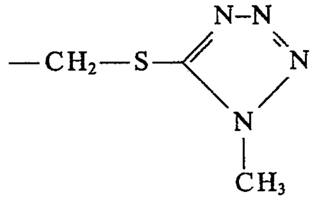

or

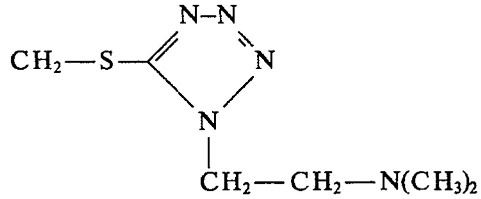

$Z_1$ denotes hydrogen or methoxy
$B_1$ denotes a radical of the formulae

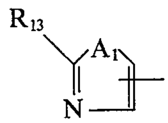
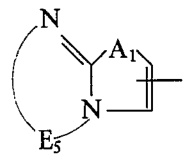

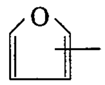 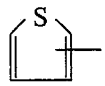 or 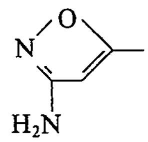

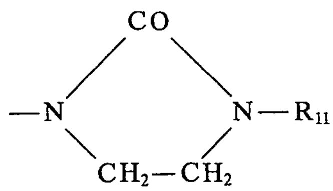

$X_5$ denotes

$R_{11}$ denotes hydrogen, methyl, ethyl, cyclopropyl, methylsulphonyl or furylideneamino,
$R_{13}$ denotes hydrogen, amino, $C_1-C_4$-alkylamino, di-$C_1-C_4$-alkylamino, $C_1-C_4$-alkylcarbonylamino, piperidino, morpholino, thiomorpholino, piperazino, 4-$C_1-C_4$-alkylpiperazino, 4-oxothiomorpholino or 4,4-dioxothiomorpholino,
$E_5$ denotes $CH_2-CH_2$, $CH_2-CH_2-CH_2$, $CH=CH$, $CH=CH-CO-CO-CH=CH$, $CO-CH_2-CH_2$, $CH_2-CH_2-NH$ or $NH-CH_2-CH_2$, it being possible for the CH and $CH_2$ radicals to be substituted by OH or $NH_2$, and
$A_1$ denotes NH, O or S.

3. β-Lactam compounds according to Claim 2 of the general formula III

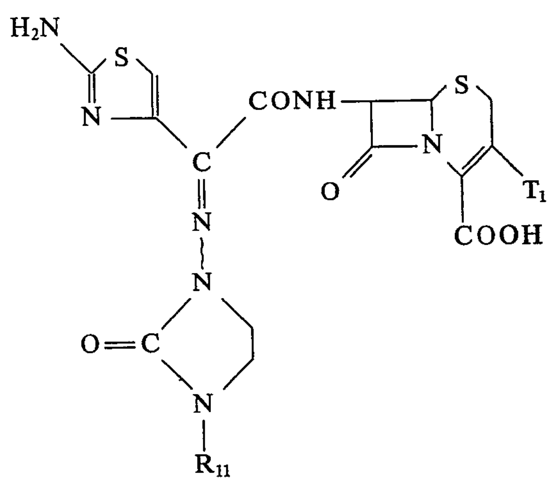
(III)

wherein $T_1$ and $R_{11}$ have the meaning indicated in Claim 2.

4. 7-[2-Amino-4-triazol-4-yl)-N-(imidazolidin-2-on-1-yl)-2-iminoacetamido]-3-acetoxymethyl-3-cephem-4-carboxylic acid of the formula

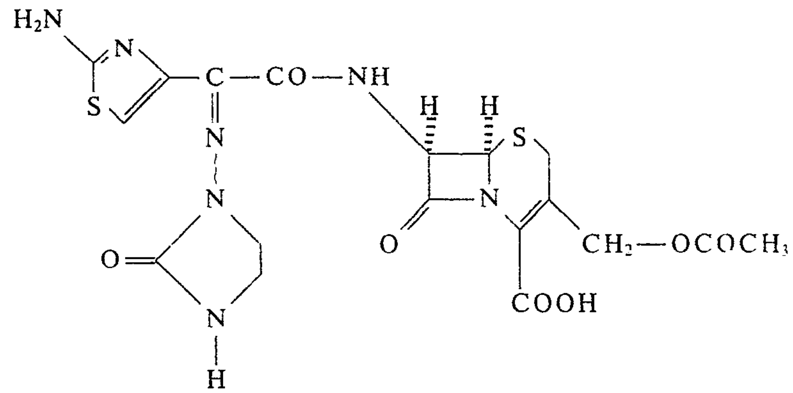

5. 7-{2-(2-Amino-4-thiazol-4-yl)-N-[(1-furylideneamino-2-oxo-imidazolidin-3-yl)-carbonylamino]-2-iminoacetamido}-3-acetoxymethyl-3-cephem-4-carboxylic acid of the formula

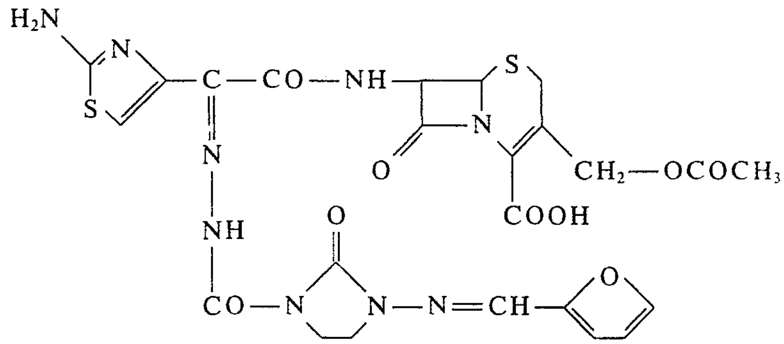

6. Process for the preparation of β-lactam compounds according to Claim 1, characterised in that compounds of the formula

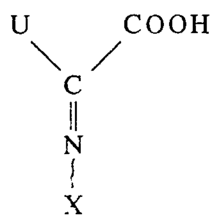

wherein

U denotes B, a group B provided by one or more protective groups or a chemical precursor of B and

B and X have the meaning indicated in Claim 1,

are reacted, after activating the carbonyl group, with compounds of the formula

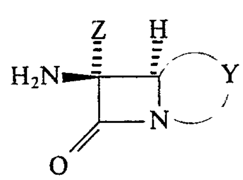

wherein Z and Y have the meaning given in Claim 1,

and, if appropriate, the protective groups are split off or the chemical precursor U is converted into B.

7. Process for the preparation of β-lactam compounds according to Claim 1, characterised in that compounds of the formula

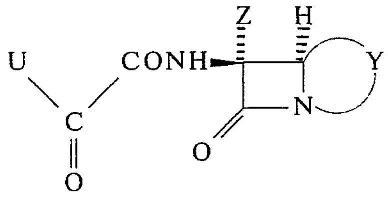

wherein

U denotes B, a group B provided by one or more protective groups or a chemical precursor of B and

Z and Y have the meaning given in Claim 1,

are reacted with compounds of the formula

$$H_2N—X$$

wherein X has the meaning given in Claim 1,

and, if appropriate, the protective groups are split off or the chemical precursor U is converted into B.

8. Medicaments, characterised in that they contain a compound according to Claim 1.

9. Process for the preparation of a medicament, characterised in that a compound according to Claim 1 is mixed with pharmaceutically suitable excipients an/or additives.

10. Feed additive, characterised in that it contains a compound according to Claim 1.

## Revendications

1. Composés du type $\beta$-lactame de formule générale I:

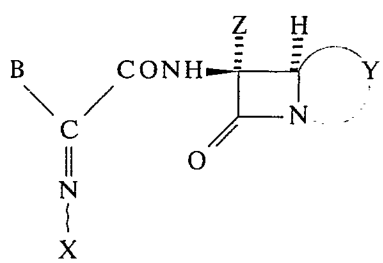

(I)

dans laquelle:

B est un noyau pentagonal ou hexagonal hétérocyclique éventuellement substitué ou un noyau phényle éventuellement substitué

Z représente H ou un groupe alkoxy en $C_1$ à $C_4$

X est un reste $-(NH-CO)_n-W$, dans lequel n est égal à 0 ou à 1 et W est un groupe 2-oxo-imidazolidin-1-yle éventuellement substitué et

Y est un reste complétant un dérivé de pénicilline ou de céphalosporine, d'isocéphem, de des-thiaoxacéphem ou d'iso-des-thiaoxacéphem.

2. Composés du type $\beta$-lactame suivant la revendication 1, de formule générale II:

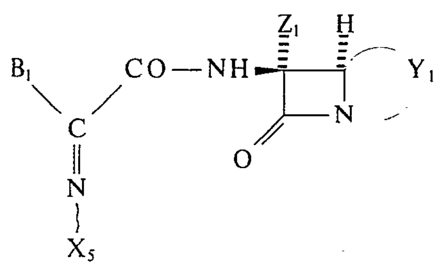

(II)

dans laquelle:

$Y_1$ désigne, sous la forme de l'acide libre, un reste des formules:

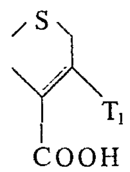

ou

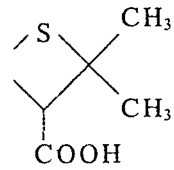

$T_1$ représente:

$CH_2—O—CO—CH_3$, $—CH_2—O—CO—NH_2$,

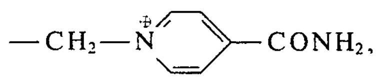

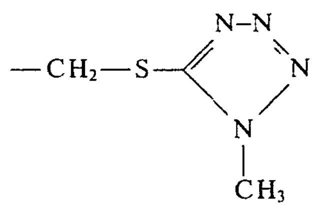

on

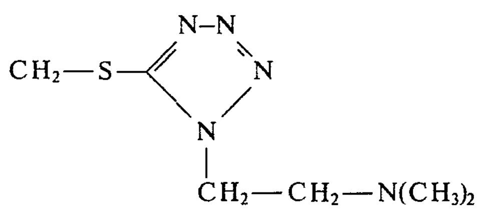

$Z_1$ est l'hydrogène ou un groupe méthoxy
$B_1$ est un reste des formules:

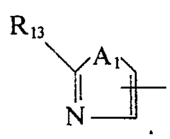

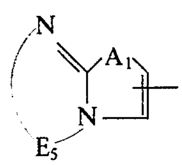

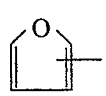

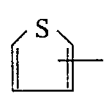

ou

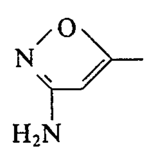

$X_5$ représente:

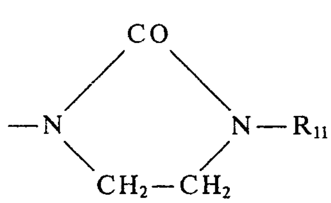

$R_{11}$ est l'hydrogène, un groupe méthyle, éthyle, cyclopropyle, méthylsulfonyle ou furylidèneamino
$R_{13}$ est l'hydrogène, un groupe amino, (alkyle en $C_1$ à $C_4$)-amino, di-(alkyle en $C_1$ à $C_4$)-amino, (alkyle en $C_1$ à $C_4$)-carbonylamino, pipéridino, morpholino, thiomorpholino, pipérazino, 4-(alkyle en $C_1$ à $C_4$)-pipérazino, 4-oxothiomorpholino ou 4,4-dioxothiomorpholino
$E_5$ représente $CH_2—CH_2$, $CH_2—CH_2—CH_2$, $CH=CH$, $CH=CH—CO—CO—CH=CH$, $CO—CH_2—CH_2$, $CH_2—CH_2—NH$ ou $NH—CH_2—CH_2$, les restes CH- et $CH_2$- pouvant être substitués par un radical OH ou $NH_2$, et
$A_1$ désigne NH, O ou S.

3. Composés du type $\beta$-lactame suivant la revendication 2, de formule générale III:

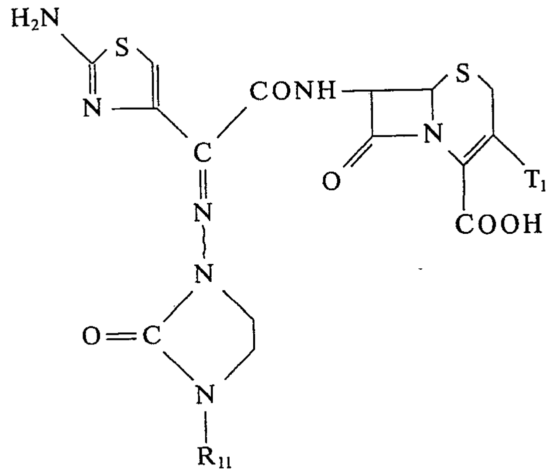

(III)

dans laquelle $T_1$, $R_{11}$ ont la définition donnée dans la revendication 2.

4. L'acide 7-(2-amino-4-thiazol-4-yl)-N-(imidazolidine-2-one-1-yi)-2-imino-acétamido)-3-acétoxy-méthyl-3-céphem-4-carboxylique de formule:

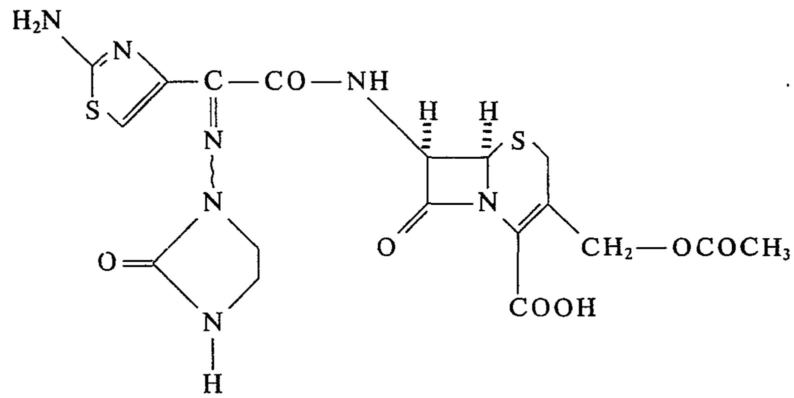

5. L'acide 7-{2-(2-amino-4-thiazol-4-yl)-N-[(1-furylidène-amino-2-oxo-imidazolidin-3-yl)-carbonyl-amino]-2-imino-acétamido}-3-acétoxyméthyl-3-céphem-4-carboxylique de formule:

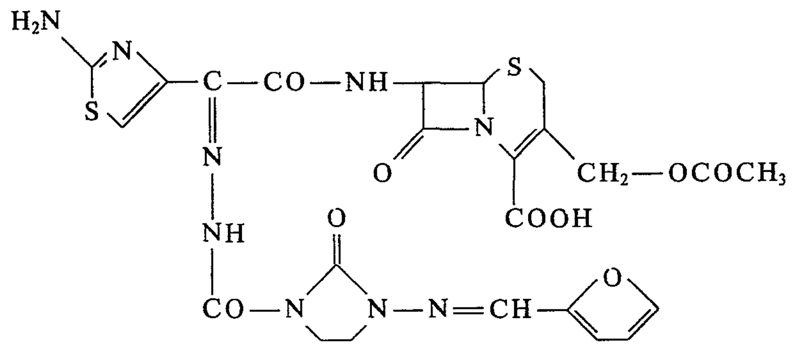

6. Procédé de production de composés du type $\beta$-lactame suivant la revendication 1, caractérisé en ce qu'on fait réagir des composés de formule:

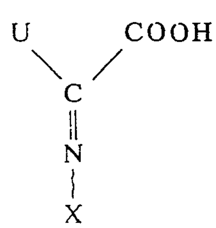

dans laquelle

U désigne B, un groupe B portant un ou plusieurs groupes protecteurs ou un progéniteur chimique de B et
B et X ont la définition donnée dans la revendication 1,

après activation du groupe carbonyle, avec des composés de formule:

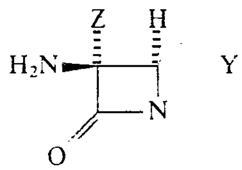

dans laquelle Z et Y ont la définition donnée dans la revendication 1 et on élimine éventuellement les groupes protecteurs ou on transforme le progéniteur chimique U en B.

7. Procédé de production de composés du type $\beta$-lactame suivant la revendication 1, caractérisé en ce qu'on fait réagir des composés de formule:

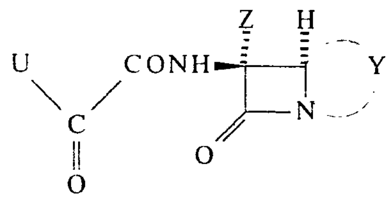

dans laquelle

U représente B, un groupe B portant un ou plusieurs groupes protecteurs ou un progéniteur chimique de B et
Z et Y ont la définition donnée dans la revendication 1,

avec des composés de formule:

$$H_2N—X$$

dans laquelle X a la définition donnée dans la revendication 1,

et on élimine éventuellement les groupes protecteurs ou on transforme le progéniteur chimique U en B.

8. Médicament, caractérisé par une teneur en un composé suivant la revendication 1

9. Procédé de production d'un médicament, caractérisé en ce qu'on mélange un composé suivant la revendication 1 avec des supports et/ou des additifs acceptables du point de vue pharmaceutique.

10. Additif alimentaire, caractérisé par une teneur en un composé suivant la revendication 1.